# EUROPEAN PATENT APPLICATION

(11) **EP 2 014 281 A1**
(43) Date of publication of application: **14.01.2009**
(21) Application number: 07301130.6
(22) Date of filing: 19.06.2007
(51) Int. Cl.: A61K 31/00, A61K 31/166, A61P 43/00, A61P 21/00

(54) **Use of inhibitors of sirtuins and/or ampk for the preparation of a medicament for the treatment of polyalanine diseases.**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Warcoin, Jacques

(57) **Abstract**

This invention relates to the use of inhibitors of sirtuins and/or AMPK for the preparation of a medicament for the treatment of polyalanine diseases, in particular oculopharyngeal muscular dystrophy (OPMD). The invention also relates to methods of identifying compounds which can be useful for treating said diseases.

## Description

### Background of the Invention

### Field of the invention

This invention relates to the use of inhibitors of sirtuins and/or AMPK for the preparation of a medicament for the treatment of polyalanine diseases, in particular oculopharyngeal muscular dystrophy (OPMD). The invention also relates to methods of identifying compounds which can be useful for treating said diseases.

### Description of prior art

Polyalanines expansion is a mutation involved in various human diseases, like for example OPMD or other genetics diseases but also psychiatric diseases, neurodegeneratives diseases.

In addition, proteins containing long polyalanine stretches are present in Huntington's disease, as a result of the frameshifting of the original CAG/polyglutamine protein. Expression of long polyalanine repeats in flies leads to toxicity and aggregation, comparable to that observed in the human disease caused by these polyalanine expansions.

A particular polyalanine disease is the myopathy OPMD, an adult-onset disease with a world-wide distribution caused by expanded polyalanines (polyAlas) in polyadenosine binding protein nuclear 1 (PABPN1).

Normal PABPN1 contains 10 Alanines (Alas), expanded to 11-17 (most frequently 13) Alas. PABPN1 may be involved in RNA polyadenylation, active transport of poly(A) mRNAs, and control of gene expression in muscles. Mechanisms for muscle cell decline/survival in OPMD pathogenesis are unknown.

Mechanisms for mutant PABPN1 (mt-PABPN1) cytotoxicity are unknown.

PABPN1 is normally found in the nucleoplasm of muscle cells, forming NIs in muscle fiber nuclei.

Mutant PABPN1 (mt-PABPN1) has been proposed to induce nuclear inclusions (NI) formation and may impair molecular chaperones and members of the ubiquitin-proteasome pathway. However, the link between NIs and pathogenicity is unclear. The aggregation of PABPN1 may primarily involve oligomerization domains in the protein, and the expanded polyAlas may be dispensable for NI formation as OPMD-like NIs are detected in normal rat tissues. Additionally, live microscopy has revealed that cells harboring an increased amount of soluble mt-PABPN1 are more prone to death than those with NIs. This suggests that, while RNAs and proteins may be sequestered in NIs, which may disturb cell homeostasis, the soluble to weakly-aggregated mutant PABPN1 may be the primary cause for cytotoxicity in OPMD. This is consistent with the notion that the cytotoxicity of aggregate-prone proteins is significantly reduced by macroautophagy inducers like rapamycin. Survival mechanisms that may be altered by mutant PABPN1 localized outside the NIs are unknown. Besides muscle-specific pathways, core modulators of stress response may be important to muscle cell survival processes.

OMPD is characterized clinically by progressive swallowing difficulties (dysphagia), eyelid drooping (ptosis) and proximal limb weakness after the age of 50. Unique nuclear filament inclusions in skeletal muscle fibers are its pathological hallmark. OPMD is characterized by nuclear inclusions (NIs) and muscle cell loss.

It would be highly desirable to be provided with a tool for the prevention or the treatment of a disease related with polyalanine accumulation in the nucleus, such as observed in OPMD.

To investigate the modulators of mutant PABPN1 cytotoxicity in a simple in vivo model, the inventors generated *C*. *elegans* transgenics expressing human PABPN1 with 0, 10, or 13 Alas. Animals stably expressing nuclear GFP under the control of the minor myosin heavy chain gene *myo-3 (Pmyo-3)* were engineered to express integrated arrays encoding PABPN1 in muscle cells using the same promoter. The transgenic phenotypes observed comprised adult-stage abnormalities, notably motility defects and muscle cell degeneration produced by mt-PABPN1 and not by normal PABPN 1 expression.

In muscle cells, PABPN1 is associated with SKIP, a protein that binds to the oncoprotein SKI in a complex that regulates transcription. SKIP is a conserved protein that interacts with members of the histone deacetylase (HDAC) complex.

HDACs are members of an ancient enzyme family found in animals, plants, fungi and bacteria. It is thought that HDACs evolved in the absence of histone proteins. Indeed, eukaryotic HDACs can deacetylate non-histone as well as histone substrates, and some HDACs reside in the cytoplasm (where histones are synthesized and acetylated for proper assembly, without the intervention of HDACs) and in mitochondria (where histones are absent).

Four HDAC classes have been identified. In particular the inventors have focused on class 3 or the so-called sirtuins, from the yeast protein Sir2 which uses NAD as a cosubstrate (Imai et al., 2000; Moazed, 2001; Smith et al., 2000; Tanner et al., 2000; Tanny and Moazed, 2001). Sir2 (whose homolog in mammals is known as SIRT1, SIR2L1 or Sir2, SIRT2, SIRT3, SIRT4, SIRT5, SIRT6 and SIRT7) is the namesake of a family of closely related enzymes, the sirtuins.

Seven sirtuins are known in mammals.
- SIRT1 (also known as Sir2α. Accession number in Pubmed NP_036370) is one the best mammal homolog of Sir2 together with SIRT2 and SIRT3.
   Mice that overexpress SIRT1 show eight properties of calorie restriction, including low cholesterol, low blood glucose, and low insulin levels. They also show increased numbers of mitochondria in their neurons.
- SIRT2 (Accession number in is active in the nucleolus, a compartment of the nucleus reserved for the assembly of ribosomes, the makers of proteins.

Sir2 is short for Silent mating type Information Regulation-2 and sirtuin stands for Sir2-homolog. The name Sir2 is used for the enzyme in the yeast *Saccharomyces cerevisiae* (where it was first discovered), in the fruit fly *Drosophila melanogaster* and in the roundworm *Caenorhabditis elegans.*
The various sirtuins in mammals are named SIRT1, SIRT2, SIRT3 etc. and SIRT1 is the best mammalian homolog (sometimes claimed to be ortholog) of Sir2.
Mammalian Sir2 homologs have NAD-dependent histone deacetylase activity (Imai et al., 2000; Smith et al., 2000). These enzymes utilize NAD⁺ as a substrate to catalyze deacetylation of specific acetylated-protein substrates. Thus these class III HDACs are very sensible to cellular homeostasis and regulate more specifically longevity and survival of cells by acting on the intracellular pathway.

Deacetylation of acetyl-lysine by Sir2 is tightly coupled to NAD hydrolysis, producing nicotinamide and a novel acetyl-ADP ribose compound. The NAD-dependent deacetylase activity of Sir2 is essential for its functions which can connect its biological role with cellular metabolism in yeast.

Biochemical studies have shown that Sir2 can readily deacetylate the amino-terminal tails of histones H3 and H4, resulting in the formation of 1-O-acetyl-ADP-ribose and nicotinamide. Strains with additional copies of SIR2 display increased rDNA silencing and a 30% longer life span. It has recently been shown that additional copies of the *C*. *elegans* SIR2 homolog, sir-2.1, and the *D. melanogaster* dSir2 gene greatly extend life span in those organisms. This implies that the SIR2-dependent regulatory pathway for aging arose early in evolution and has been well conserved. Today, Sir2 genes are believed to have evolved to enhance an organism's health and stress resistance to increase its chance of surviving adversity.

In prior art, it has been showed that neuronal dysfunction was genetically rescued by increased sirtuins dosage, cell protection, in particular neuroprotection in Huntington models, was achieved by chemical sirtuin activators as Resveratrol, (Parker et al. 2005), prevention of Alzheimer disease amyloid neuropathology may be possible by promotion of the NAD+ dependent sirtuin, SIRT1 (Quin et al. 2006), and protection against metabolic disease is possible by activating SIRT-1 by Resveratrol (Lagouge et al. 2006).

Resveratrol is a substance which experiments have shown to have a number of life-extending and health benefits in various species; it also increases the activity of Sir2 and this is the postulated reason for its beneficial effects. Resveratrol is also known to be able to activate AMPK in neurons (Dasgupta B, Milbrandt J. Proc Natl Acad Sci USA. 2007 ; 104:7217-22)

Surprisingly, inventors show in the present patent application that contrary to the existing prejudice, based on abundant prior art as previously cited, activators of Sirtuin in particular Resveratrol fragilize OPMD muscle, and was detrimental through *sir-2.1, aak-2* but not *daf-16* activity. Moreover, increased dosage of the *class III* deacetylase *sir*-*2.1*/SIRT1 exacerbated muscle pathology through the activity of the transcription factor *daf-16*/FoxO and fuel sensor *aak-2*/AMPK, while null mutants of the *sir-2.1, daf-16* and *aak-2* were protective. Finally, mt-PABPN1 cytotoxicity was enhanced by Resveratrol in mammalian cells.

Additionally, inventors show that, to the contrary, inhibitors of Sirtuins, in particular splitomicin and sirtinol, rescued the muscle pathology in nematodes and histone H4 hypoacetylation in mammalian cells,
Consistently, the Sir2 inhibitor sirtinol was protective through *sir-2.1, daf-16* but not *aak-2* activity while null mutants of the *sir-2.1, daf-16* and *aak-2* were protective.

5'AMP-activated protein kinase or AMPK (accession number in Pubmed: N°AAB32732, Beri et al. 1994) consists of three proteins (subunits) that together make a functional enzyme, conserved from yeast to humans, that plays a role in cellular energy homeostasis.

AMPK are known in prior art to act as a metabolic master switch regulating several intracellular systems including the cellular uptake of glucose, the β-oxidation of fatty acids and the biogenesis of glucose transporter 4 (GLUT4) and mitochondria. The energy-sensing capability of AMPK can be attributed to its ability to detect and react to fluctuations in the AMP: ATP ratio that take place during rest and exercise (muscle stimulation). During muscle stimulation, AMP increases while ATP decreases, which changes AMPK into a good substrate for activation via an upstream kinase complex, AMPKK. AMPKK is a complex of three proteins, STE-related adaptor (STRAD), mouse protein 25 (MO25), and LKB1 (a serine/threonine kinase). During a bout of exercise, AMPK activity increases while the muscle cell experiences metabolic stress brought about by an extreme cellular demand for ATP. Upon activation, AMPK increases cellular energy levels by inhibiting anabolic energy consuming pathways (fatty acid synthesis, protein synthesis, etc.) and stimulating energy producing, catabolic pathways (fatty acid oxidation, glucose transport, etc.). Towler MC, Hardie DG. Circ Res. 2007 Feb 16; 100 (3):328-41; Thomson DM, Porter BB, Tall JH, Kim HJ, Barrow JR, Winder WW. Skeletal Muscle and Heart LKB1 Deficiency Causes Decreased Voluntary Running and Reduced Muscle Mitochondrial Marker Enzyme Expression in Mice. Am. J Physiol Endocrinol Meta. [Epub ahead of print], 2006; Ojuka EO. Role of calcium and AMP kinase in the regulation of mitochondrial biogenesis and GLUT4 levels in muscle. Proc Nutr Soc. 63: 275-278, 2004; Winder WW, Hardie DG. AMP-activated protein kinase, a metabolic master switch: possible roles in type 2 diabetes. Am. J Physiol. 277: 1-10, 1999.

### Summary of the invention

The present invention provides therapeutic strategy to muscle protection in OPMD, in particular elicit FoxO-dependent muscle protection in OPMD by Sirtuin and/or elicit muscle protection by AMPK inhibition.

Unlike other deacetylases, many of which are involved in gene silencing, Sir2 are described in the prior art as insensitive to class I and II histone deacetylase inhibitors like trichostatin A (TSA) (Imai et al., 2000; Landry et al., 2000a; Smith et al., 2000).

Thus according to the present invention specific class III histone deacetylase inhibitors, in particular sirtinol, spiltomicin and sodium butyrate are used for the preparation of a medicament for the treatment of polyalanine diseases.

The present invention provides the use of inhibitors of sirtuins and/or AMPK for the preparation of a medicament for the treatment or the prevention of polyalanine diseases.

According to a first embodiment, the present invention concerns the use of inhibitors of sirtuins for the preparation of a medicament for the treatment or the prevention of polyalanine diseases.

As used herein, the term "polyalanine diseases" concerns any disease which is caused or associated by the presence of expanded polyalanine tracts in proteins.
Codon reiteration disorders are caused by abnormal expansions of polyalanine tracts within the coding region of a protein. These mutations impair normal protein folding, resulting in aggregate formation in the affected tissues.

Recently, expansions of trinucleotide repeats encoding polyA tracts have been recognized as the cause of at least nine diseases through the alanine containing proteins (BROWN and BROWN, Trends Genet., vol.20, p:51, 2004).

In particular polyalanine diseases according to the present invention are caused by expanded polyalanines (polyAlas) in polyadenosine binding protein nuclear 1 (PABPN1).

Said polyalanine disease is chosen according to a preferred embodiment of the invention in a group comprising oculopharyngeal muscular dystrophy (OPMD), Huntington disease, Congenital central hypoventilation, syndactyly type V, brachydactyly-syndactyly syndrome, X linked mental retardation, X-linked hypopituitarism, Currarino syndrome, lepharophimosis/ptosis/epicanthus inversus syndrome, and schizophrenia.

Congenital central hypoventilation syndrome is caused by a PHOX2B gene mutation, novel brachydactyly-syndactyly syndrome is caused by a mutation in HOXD13 gene, X-linked hypopituitarism is caused by a mutation in SOX3 gene, Currarino syndrome is caused by a mutation in HLXB9 gene, lepharophimosis/ptosis/epicanthus inversus syndrome is caused by a mutation in FOXL2 gene, and schizophrenia is caused by a mutation in PMX2B gene.

According to the preferred embodiment of the invention, the polyalanine disease is oculopharyngeal muscular dystrophy (OPMD).

Oculopharyngeal muscular dystrophy (OPMD), is a form of muscular dystrophy characterized in some stages by deformation of the eyelid, speech impediment, and difficulty swallowing due to dystrophia of the pharynx.

According to another preferred embodiment of the invention, the polyalanine disease is Huntington disease. In particular, according to the present invention the polyalanine disease is Huntington disease associated with polyalanine expansion.

Huntington's disease is classically known to be caused by a trinucleotide repeat expansion in the Huntingtin (Htt) gene and is one of several polyglutamine (or PolyQ) diseases. This expansion produces an altered form of the Htt protein, mutant Huntingtin (mHtt), which results in neuronal cell death in select areas of the brain and is a terminal illness. It has been thought that there can be a link between diseases caused by polyglutamine and polyalanine expansion mutations as it has been shown that the expanded CAG/polyglutamine tract within the SCA3 gene can shift to the CGA/polyalanine frame. It has been shown recently that this frameshifting phenomenon is more widespread and occurs in Huntington's disease. (Polyalanine and polyserine frameshift products in Huntington's disease Davies and Rubinsztein J Med Genet.2006; 43: 893-896).

Thus, according to the present invention, Huntington disease is considered as a polyalanine disease.

Said inhibitors of sirtuins are chosen according to a preferred embodiment of the invention in a group comprising, cambinol, splitomycin, nicotinamide, suramin, NF279 and NF2030, Dihydrocoumarin, indoles, N,N'-bisbenzylidenebenzene-1,4-diamine and N,N'-bisbenzylidenenaphthalene-1,4-diamine derivatives, in particular N,N'-bis(2-hydroxybenzylidene)benzene-1,4-diamine), 5-{[(2-{[(3,5-dichloro-2-hydroxyphenyl)methylene]amino}phenyl)thio]methyl}-4-(hydroxymethyl)-2-methylpyridin-3-ol, 1,4-di[(4-hydroxyphenethyl)amino]-9,10-dihydroanthracene-9,10-dione, 2-({[3-[(2-hydroxy-3 - methoxybenzylidene)amino]-5-(trifluoromethyl)phenyl]imino}methyl)-6-methoxyphenol, 2-chloro-5,5a,6,7,8,9,10,10a-octahydro-Cyclohept[b]indole-6-carboxamide, 3,3'-[[4-[[4-[4-(5,6,7,8-tetrahydro-5,5,8,8-tetramethyl-2-naphthalenyl)-2-thiazolyl]-1-piperidinyl]methyl]phenyl]imino]bis-Propanenitrile, N-[2-(5-fluoro-1H-indol-2-yl)ethyl]-4-[[3-[4-[(4-fluorophenyl)methoxy]phenyl]-1-oxo-2-propen-1-yl]amino]-4-Piperidinecarboxamide, N-[3-[(2-ethylhexyl)oxy]propyl]-4-hydroxy-γ-(4-hydroxyphenyl)-γ-methyl-Benzenebutanamide, sirtinol, and geometrical and/or optical isomers of sirtinol, and siRNA dsRNA ou RNA sirtuins antisens.

Nicotinamide, suramin, NF279 and NF2030 are as described for example in Konrad T. Howitz et al., Nature 2003, Vol 425, 191-196 and in Schuetz A. et al., Structure 2007, Vol 15, 377-89).

The formula of the 2-chloro-5,5a,6,7,8,9,10,10a-octahydro-Cyclohept[b]indole-6-carboxamide is

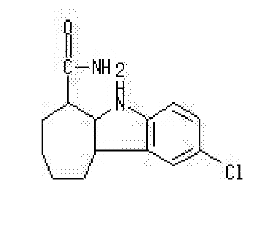

The formula of the 3,3'-[[4-[[4-[4-(5,6,7,8-tetrahydro-5,5,8,8-tetramethyl-2-naphthalenyl)-2-thiazolyl]-1-piperidinyl]methyl]phenyl]imino]bis-Propanenitrile is

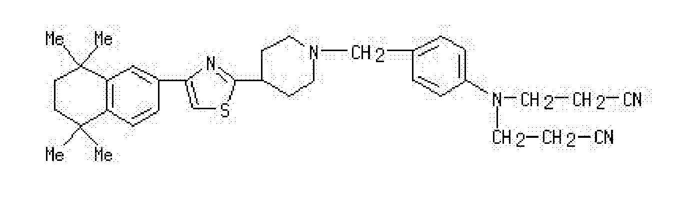

The formula of the N-[2-(5-fluoro-1H-indol-2-yl)ethyl]-4-[[3-[4-[(4-fluorophenyl)methoxy]phenyl]-1-oxo-2-propen-1-yl]amino]-4-Piperidinecarboxamide is

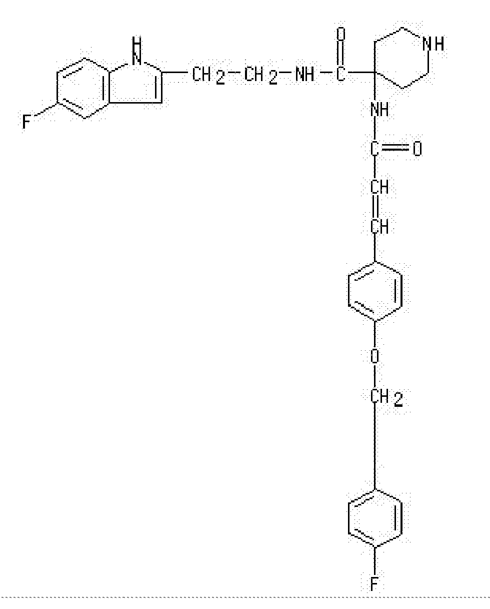

The formula of the N-[3-[(2-ethylhexyl)oxy]propyl]-4-hydroxy-γ-(4-hydroxyphenyl)-γ-methyl-Benzenebutanamide is

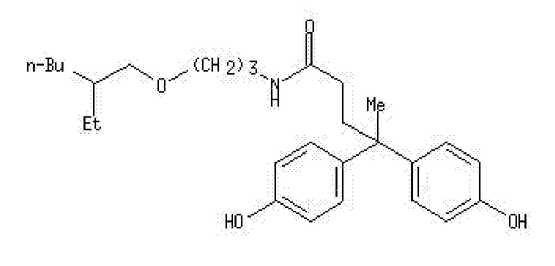

Geometrical isomers of sirtinol are 3- and 4-[(2-hydroxy-1-naphthalenylmethylene)amino]-N-(1-phenylethyl)benzamide (i.e., m- and p-sirtinol) and optical isomers of sirtinol are (R)- and (S)-sirtinol as described for example in A. Mai et al, J Med Chem 2005, Vol 48, 7789-95. According to the present invention, isomers of sirtinol are only geometrical, only optical or both geometrical and optical isomers (for example m-R sirtinol, p-S sirtrinol).

Dihydrocoumarin is as reported for example in Olaharski AJ et al., Plos Genetics 2005, Vol 1).
N,N'-bisbenzylidenebenzene-1,4-diamine and N,N'-bisbenzylidenenaphthalene-1,4-diamine derivatives (notably N,N'-bis(2-hydroxybenzylidene)benzene-1,4-diamine) are as described for example in Kirivanta et al, J Med Chem 2006, vol 49, 7907-11, and are particularly good inhibitors of SIRT2.

Indoles is as described for example in A.D. Napper et al., J Med Chem 2005, vol 48 , 8045-54, and is particularly good inhibitor of SIRT1.

siRNA dsRNA or RNA SiR2 antisens according to the present invention will inhibit Sir2 expression and activity by inducing RNA interference in cells.
RNA interference (RNAi) is a process whereby the introduction of double stranded RNA (dsRNA) into a cell inhibits gene expression post-transcriptionally, in a sequence dependent fashion. This process is also known as post-transcriptional gene silencing. Current models of RNAi indicate that it is mediated by short (typically 20-25 nucleotides) dsRNAs known as small interfering RNAs' (siRNA). It appears that dsRNA is cleaved in the cell to create siRNAs. siRNAs are then incorporated into an RNA-induced silencing complex(RISC), guiding the complex to the homologous endogenous mRNA. The activated RISC then cleaves the RNA transcript, resulting in the destruction of the RNA in a cell which is homologous to the siRNAs. The siRNAs are re-cycled. In this way, a relatively small number of siRNAs can selectively destroy a large excess of bcellular mRNA.
To induce RNA interference in a cell, dsRNA may be introduced into the cell as an isolated nucleic acid fragment or via a transgene, plasmid or virus. Alternatively, siRNA may be synthesised and introduced directly into the cell siRNA sequences are selected on the basis of their homology to the gene it is desired to silence.

Homology between two nucleotide sequences may be determined using a variety of programs including the BLAST program, of Altschul et al. (1990) J. Mol. Biol. 215: 403-10, or BestFit, which is part of the Wisconsin Package, Version 8, September 1994, (Genetics Computer Group, 575 Science Drive, Madison, Wisconsin, USA, Wisconsin 53711). Sequence comparisons may be made using FASTA and FASTP (see Pearson & Lipman, 1988. Methods in Enzymology 183: 63-98). Parameters are preferably set, using the default matrix, as follows: Gapopen (penalty for the first residue in a gap):-16 for nucleic acid; Gapext (penalty for additional residues in a gap):-4 for nucleic acids; KTUP word length: 6 for nucleic acids.

Sequence comparison may be made over the full length of the relevant sequence, or may more preferably be over a contiguous sequence of about or 10,15, 20,25 or 30 bases.

Preferably the degree of homology between the siRNA and the target gene is at least 75%, at least 80%, at least 85%, at least 90%, at least 95%, at least 97%, or at least 99%.

The degree of homology between the siRNA or dsRNA and the gene to be silenced will preferably be sufficient that the siRNA or dsRNA will hybridise to the nucleic acid of the gene sequence under stringent hybridisation conditions.

The siRNA may be between 10bp and 30bp in length, preferably between 20bp and 25bp. Preferably, the siRNA is 19, 20, 21 or 22bp in length.

The siRNA sequence may be, for example, any suitable contiguous sequence of 10-30bp from the sequence of SiRT1 (SEQ ID N°.9), SiRT2 (SEQ ID N°. 10) SiRT3 (SEQ ID N°. 11), SiRT4 (SEQ ID N°. 12), SiRT5 (SEQ ID No. 13) SiRT6 (SEQ ID No. 14). SiRT7 (SEQ ID N°: 15). Alternatively, longer dsRNA fragments comprising contiguous sequences from the sequences of SEQ ID NO. 9, SEQ ID N° 10, SEQ ID NO. 11, SEQ ID N°12 SEQ ID NO. 13, SEQ ID N° 14 or SEQ ID N° 15 may be used, as they will be cleaved to form siRNAs within the cell. Preferably, the siRNA sequence is SEQ ID N°s: 16 & 17.

According to the preferred embodiment of the invention, inhibitor of sirtuins is sirtinol or p-sitinol.
Sirtinol is also known as "Sir Two Inhibitor Naphthol" or "2-[(2-Hydroxynaphthalen-1-yl-methylene)amino]-N-(1-phenethyl)benzamide". Sirtinol is a specific cell-permeable inhibitor of the sirtuin family of NAD-dependent deacetylases with no effect on human HDAC1.

According to another preferred embodiment of the invention, inhibitor of sirtuins is Splitomycin. Splitomycin is also know as 1,2-Dihydro-3H-naphtho[2,1-b]pyran-3-one.

According to a second embodiment, the present invention concerns the use of inhibitors of AMPK for the preparation of a medicament for the treatment or the prevention of polyalanine diseases.

By "AMPK inhibitor", it is meant herein a compound which inhibits AMPK as determined by the method described by Witters, et al., Journal of Biological Chemistry, 267, pp.2864-2867 (1992).

Said inhibitor of AMPK is chosen according to a preferred embodiment of the invention in a group comprising Compound C and indirubin-3'-oxime, in particular, inhibitor of AMPK is Compound C.

By "compound C" it is meant 6-[4-(2-Piperidin-1-yl-ethoxy)-phenyl)]-3-pyridin-4-yl-pyrrazolo [1,5-a]-pyrimidine.

The medicament of the present invention can be presented for administration to humans, mammals and other animals in unit dosage forms, such as tablets, capsules, pills, powders, granules, sterile parenteral solutions or suspensions, oral solutions or suspensions, oil in water and water in oil emulsions containing suitable quantities of the compound, suppositories and in fluid suspensions or solutions.

For oral administration, either solid or fluid unit dosage forms can be prepared. For preparing solid medicaments such as tablets, the compound can be mixed with conventional ingredients such as talc, magnesium stearate,dicalcium phosphate, magnesium aluminum silicate, calcium sulfate, starch, lactose, acacia, methylcellulose and functionally similar materials as pharmaceutical diluents or carriers. Capsules are prepared by mixing the compound with an inert pharmaceutical diluent and filling the mixture into a hard gelatin capsule of appropriate size. Soft gelatin capsules are prepared by machine encapsulation of a slurry of the compound with an acceptable vegetable oil, light liquid petrolatum or other inert oil.

Fluid unit dosage forms or oral administration such as syrups, elixirs, and suspensions can be prepared. The forms can be dissolved in an aqueous vehicle together with sugar, aromatic flavoring agents and preservatives to form a syrup. Suspensions can be prepared with an aqueous vehicle with the aid of a suspending agent such as acacia, tragacanth, methylcellulose and the like.

For parenteral administration fluid unit dosage forms can be prepared utilizing the compound and a sterile vehicle. In preparing solutions the compound can be dissolved in water for injection and filter sterilized before filling into a suitable vial or ampoule and sealing. Adjuvants such as a local anesthetic, preservative and buffering agents can be dissolved in the vehicle. The medicament can be frozen after filling into a vial and the water removed under vacuum. The lyophilized powder can then be scaled in the vial and reconstituted prior to use.

According to a third embodiment, the present invention concerns the use of AMPK activators like for example but not only AICAR for the preparation of a medicament for the treatment or prevention of polyglutamine diseases like for example but not restricted to, Huntington's disease. The inventors have indeed observed that a null mutation in aak-2/AMPK aggravates the cytotoxicity of mutant polyglutamines as expressed in transgenic nematodes as part of a N-terminal fragment of human huntington (The Huntington's disease protein). These transgenic nematodes have been previously described (Parker et al. PNAS 2001).

According to a fourth embodiment, the present invention concerns a method of identifying a compound which can be useful for the treatment of polyalanine diseases, the method comprising the following steps:
(i) administering a candidate compound inhibitors of sirtuins and/or AMPK to transgenic invertebrate organisms expressing human PABPN1 with 13 Alanines.
(ii) comparing the motility and/or survival of muscle cells of said transgenic invertebrate organisms before and after step (i)
(iii) identifying as compound which can be useful for the treatment of polyalanine diseases compounds that enhances the motility and /or the survival of muscle cells of said transgenic invertebrate organisms.

In particular, this method permits to select a compound between different candidate compounds inhibitors of sirtuins and/or AMPK which can be useful for the treatment of polyalanine diseases according to the present invention with the best activity in rescuing muscle, in particular OPMD muscle, and/ or enhancing the motility and /or the survival of muscle cells but also the less toxicity on said transgenic invertebrate organisms.

Optionally, the method according to the invention comprises additional step (i ') of administering a candidate compound inhibitors of sirtuins and/or AMPK to transgenic invertebrate organisms expressing human PABPN1 with 10 Alanines as a control.

According to a fifth embodiment, the present invention concerns a method of identifying a compound which can be useful for the treatment of polyalanine diseases, the method comprising the following steps:
(i) administering a candidate compound inhibitors of sirtuins and/or AMPK to transgenic invertebrate organisms expressing human PABPN1 with 13 Alanines and to transgenic invertebrate organisms expressing human PABPN1 with 10 Alanines.
(ii) comparing the motility and/or survival of muscle cells of transgenic invertebrate organisms expressing human PABPN1 with 13 Alanines, with transgenic invertebrate organisms expressing human PABPN1 with 10 Alanines.
(iii) identifying as compound which can be useful for the treatment of polyalanine diseases compounds that reduces or suppress differences of motility and /or survival of muscle cells of transgenic invertebrate organisms expressing human PABPN1 with 13 Alanines, compared to transgenic invertebrate organisms expressing human PABPN1 with 10 Alanines.

In particular, this method permits to select a compound between different candidate compounds inhibitors of sirtuins and/or AMPK which can be useful for the treatment of polyalanine diseases according to the present invention with the best activity in rescuing muscle, in particular OPMD muscle, and/ or enhancing the motility and /or the survival of muscle cells but also the less toxicity on said transgenic invertebrate organisms.

Moreover an another method of identifying a compound which can be useful for the treatment of polyalanine diseases according to the present invention is to do a screening of candidate compound inhibitors of sirtuins and/or AMPK on mammal cells expressing mutated PABPN1, in particular fibroblast or muscular fibers in culture.

### DESCRIPTION OF THE DRAWINGS

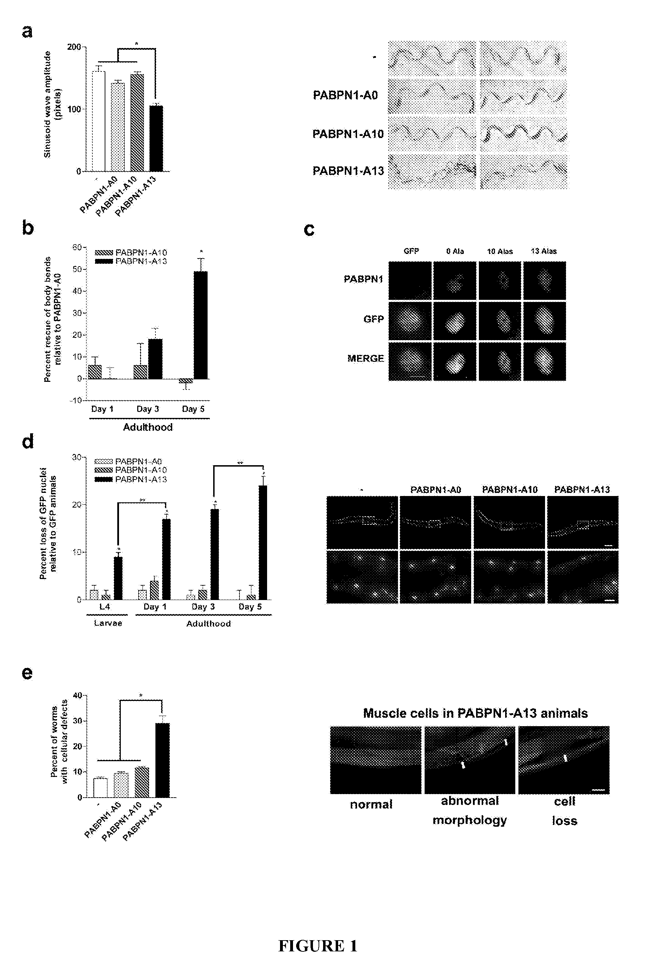

**Figure 1****.** Mutant PABPN1 produces muscle pathology in adult *C. elegans,* **a.** Abnormal motility induced by mutant PABPN1 is shown as the amplitude of the sinusoid wave (mean±SEM). **P* < 0.001 compared to each of the controls. The right panel shows representative images of the sinusoid waves. **b**. Abnormal motility induced by mutant PABPN1 is shown as the percentage of number of total body bends (mean±SEM) compared to PABPN1-AO animals. **P* < 0.001 compared to PABPN1-A10. **c.** Expression of PABPN1 in muscle cell nuclei. Staining of whole worms with a human PABPN1 antibody (in red) revealed a diffuse expression of the protein in GFP-positive nuclei of PABPN1-A0 and PABPN1-A10 and a diffuse to weakly-aggregated expression of mutant PABPN1. Scale bar is 5 µm. **d.** Age-dependent loss of muscle cell nuclei induced by mutant PABPN1. The left panel shows the percent loss (mean±SEM) in comparison to GFP animals. **P* < 0.001 compared to PABPN1-A10. ***P* < 0.001 compared to as indicated. The right panel shows GFP signals in representative young adults (upper panels: magnification is 10X and scale bar 100 µm) and corresponding body wall muscle nuclei (lower panels: magnification is 63X and scale bar 20 µm). **e.** Muscle cell degeneration induced by mutant PABPN1. The left panel shows the percentage of worms with abnormal or missing muscle cells as visualized by staining of actin filaments. **P* < 0.001 compared to each of the controls. The right panel shows staining of representative muscular cells in PABPN1-A13 animals (scale bar is 20 µm).

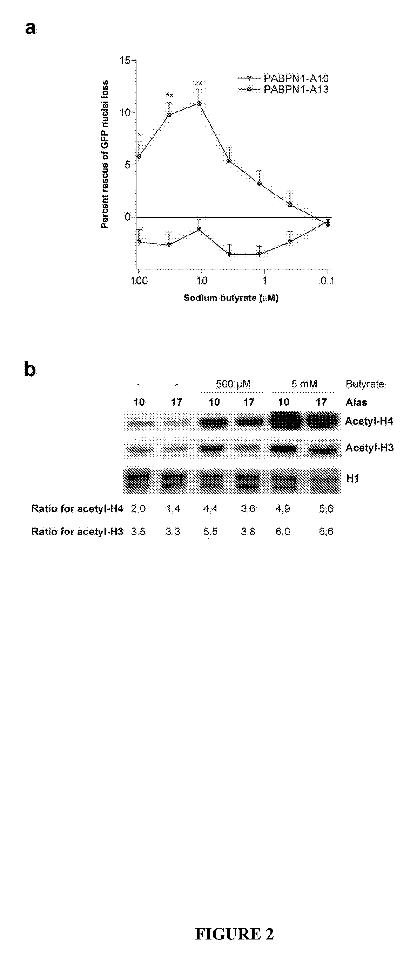

**Figure 2****.** Sodium butyrate rescues muscle pathology in nematodes and H4 hypoacetylation in mammalian cells. **a.** Sodium butyrate rescues the loss of muscle cell nuclei induced by mutant PABPN1 in *C.elegans.* Protection is shown as percent rescue versus untreated controls. ***P* < 0.001 and **P* <0.05 compared to untreated controls. **b**. A reduction in acetylation of histone H4 is induced by mutant PABPN1 expression in mammalian cell culture. COS-7 cells expressing mutant PABPN1 with 17 Alas showed a 29.7±0.4 % *(P* < 0.01 compared to PABPN1-A10) reduction in the level of acetylation of histone H4 compared to cells expressing normal PABPN1 with 10 Alas. The level of acetyl H3 was not significantly influenced by PABPN1-A17. Incubation with 500 µM or 5 mM sodium butyrate increased the level of acetylation of histone H4 in cells expressing normal or mutant PABPN1, an effect observed at lower intensity for histone H3 compared to histone H4. Equivalent levels of histones are shown with anti-histone H1.

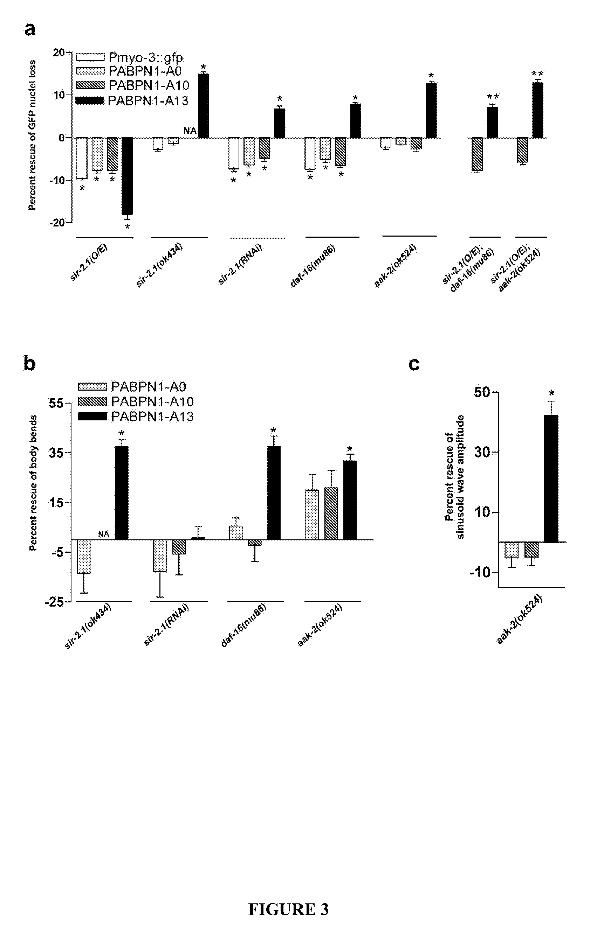

**Figure 3****.** Effects of genetic manipulations of sir-2.1, daf-16/FoxO and aak-2/AMPK on mutant PABPN1 cytoxicity. **a.** The muscle nuclei loss (MNL) induced by mutant PABPN1 was enhanced by increased sir-2.1 dosage (O/E) and inhibited by (i) a null mutation (ok434) in or gene knock-down (using RNAi; see decreased SIR-2.1 expression in supplemental figure 1) of sir-2.1, (ii) a null mutation (mu86) in daf-16/FOXO and (iii) a null mutation (ok524) in aak-2/AMPK. The PABPN1-A10; sir-2.1(ok434) animals are not available (NA) as genetic engineering was repeatedly unsuccessful, likely because the transgenic array is integrated near the sir-2.1 locus. The sir-2.1(O/E) and daf-16(mu86) mutations and the sir-2.1 RNAi similarly enhanced MNL in GFP, PABPN1-A0 and PABPN1-A10 animals in a non specific manner. The enhancement of MNL by increased sir-2.1 dosage (O/E) in mutant PABPN1 animals was reversed by the null mutations mu86 in daf-16/FOXO and ok524 in aak-2/AMPK, with no effect on MNL produced by increased sir-2.1 dosage in control PABPN1-A10 animals. The maximal achievable rescue in PAPBN1-A13 animals is 28 %. *P < 0.001 compared to control transgenics. **P < 0.001 compared to transgenics with increased sir-2.1 dosage (O/E). **b.** A null mutation of sir-2.1 (ok434), daf-16 (mu86) and aak-2 (ok524) strongly rescued the abnormal frequency of body bends caused by PABPN1-A13 expression. The maximal achievable rescue in PAPBN1-A13 animals is 37 %. The gene knock-down (RNAi) of sir-2.1 showed no effect. *P < 0.001 compared to control transgenics. **c.** A null mutation of aak-2 (ok524) fully rescued the abnormal sinusoid wave amplitude caused by PABPN1-A13 expression (maximal achievable rescue: 53 %). *P < 0.001 compared to PABPN1-A13 .

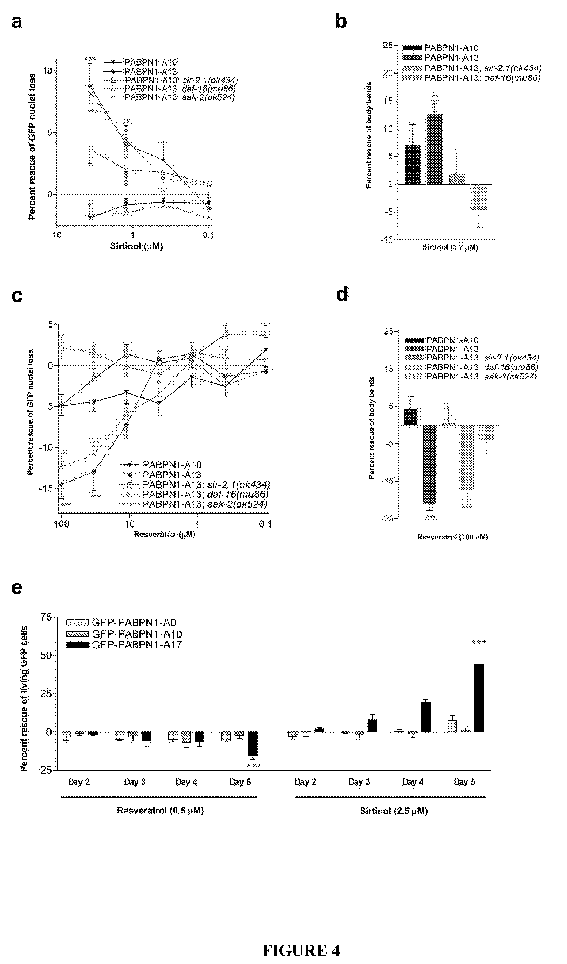

**Figure 4****.** Sirtinol and resveratrol show similar profiles of activity in C. elegans and mammalian cells expressing mutant PABPN1. **a.** Sirtinol protected against muscle nuclei loss (MNL) in *C. elegans,* an effect lost in the presence of a null mutation of sir-2.1 (ok434), daf-16 (mu86) but not aak-2 (ok524). ***P < 0.001 and *P < 0.05 compared to untreated controls. **b.** Sirtinol rescued the abnormal frequency of body bends produced by mutant PABPN1 at day 5 of adulthood in a sir-2.1 and daf-16 dependent manner. **P < 0.01 compared to untreated controls. **c.** Resveratrol enhanced MNL in *C*. *elegans,* an effect lost in the presence of a null mutation of sir-2.1 (ok434), aak-2 (ok524) and not daf-16 (mu86). ***P < 0.001 compared to untreated controls. **d.** Resveratrol enhanced the abnormal frequency of body bends produced by mutant PABPN1 at day 3 of adulthood, an effect dependent on sir-2.1, aak-2 and not daf-16. ***P < 0.001 compared to untreated controls. **e.** Resveratrol reduced and sirtinol rescued the survival of COS-7 cells expressing mutant PABPN1 fused to GFP. ***P < 0.001 compared to untreated cells.

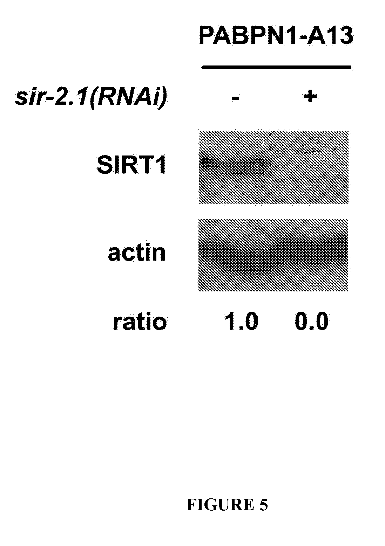

**Figure 5****.** The gene knock-down by sir-2.1 RNAi reduces SIR-2.1 expression in mutant PABPN1 animals.

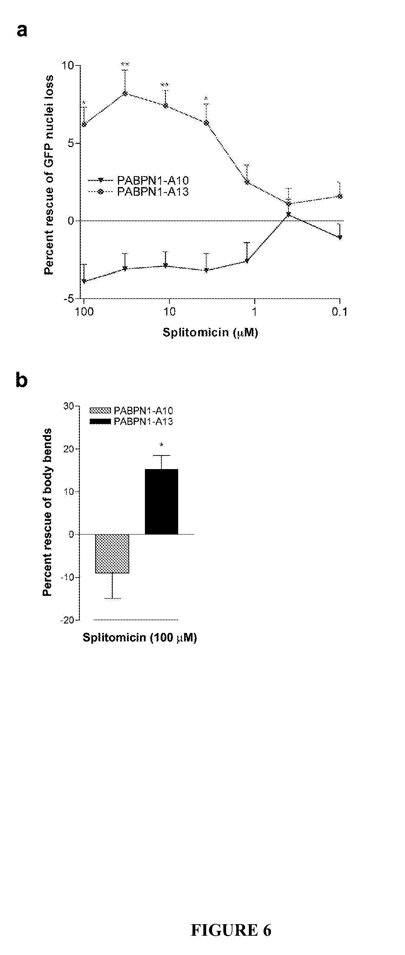

**Figure 6****.** The Sir2 inhibitor splitomicin rescues mutant PABPN1 Cytotoxicity. **a.** Splitomicin protected against muscle cell nuclei loss in *C. elegans.* *P < 0.01 and **P < 0.001 compared to untreated controls. **b.** Splitomicin rescued the abnormal motility produced by mutant PABPN1 at day 5 of adulthood. *P < 0.01 compared to untreated controls.

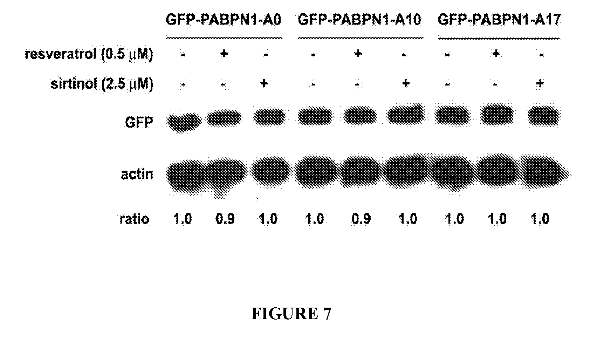

**Figure 7****.** Expression of PABPN1::GFP in COS-7 cells. Expression of PABPN1 fused to GFP is unchanged by resveratrol and sirtinol treatments. No difference in expression levels was detected by western-blotting with a GFP antibody. Equivalent levels of proteins are shown with an actin antibody.

### EXAMPLES

### METHODS

### C. elegans

**Reporter constructs.** Using PABPN1 sequence (SEQ ID N° 1) with 10, 13 Alas, we constructed *Pmyo-3::*PABPN1 (with 0, 10, and 13 Alas) GFP fusions conducted through PCR and subcloning into the *Eco*RI sites of the *myo-3::GFP* vector pPD118.20. Primers used for amplification of 10 and 13 Alas were : 5'-3' (pabF) CGGGAATTCATGGCGGCGGCGGCGGCGGCG, (SEQ ID N°2) and (pabR) CGGGAATTCTTAGTAAGGGGAATACCATGATGTTGTCGC (SEQ ID N°3). We used an alternate forward primer to amplify PABPN1 with 0 Ala (pab0F) CGGGAATTCATGGG-GGCTGCGG (SEQ ID N°4). *Pmyo-3::*PABPN1 (with 0, 10, and 13 Alas) was constructed, conducted through removal of GFP with digestion using XbaI and BtgZI enzymes. All constructs were sequenced to verify integrity.

**Genetic manipulations.** Nematode strains were received from the *Caenorhabditis elegans* Genetics Center CGC unless indicated otherwise and were maintained following standard methods. Transgenic animals were generated by standard transformation techniques¹⁸. DNA encoding PABPN1 was coinjected with a wild-type *lin-15* marker plasmid at a concentration of 50 ng/µl each into the gonads of young, adult *lin-15(n765);* ccIs4251 hermaphrodites stably expressing nuclear GFP. To test for transgene expression, RNA from L4 larvae-young adults were extracted using the RNeasy mini kit. RNAs were dosed using a Nanodrop ND-1000 and quality checked by migration on agarose gel. 100ng of total RNA were used for the RT-PCR. Quantitative PCR was performed using SYBRGreen® (ABgene) with the ABI PRISM® 7700 Sequence Detection system (Applied Biosystems). The primers were (PABPN1-F) GGAGCTGGAAGCTATCAAAGC (SEQ ID N° 5) and (PABPN1-R) CTGTTGCACCATAGTCCACATT (SEQ ID N°6) for amplification of PABPN1, and (col-1-F) CATCTTTGCTGAGGTCAACTA (SEQ ID N° 7) and (col-1-R)CATCCCTCGCATTGGAGATT (SEQ ID N° 8) for amplification of col-1. Mutant strains were first crossed with *lin-15(n765)* males. These strains were then crossed with our PABPN1 integrated strains rescuing the *lin-15 (n765)* phenotype. The mutants used in this study include *geIn3(sir-2.1(O*/*E), sir-2.1(ok434), daf-16(mu86)* and *aak-2(ok524).* To perform RNAi experiments, E. *coli* HT115 pL4440-*coq* strains were grown selectively to OD₆₆₀= 0.4 in LB medium, supplemented with 100 µg/ml ampicillin, 10 µg/ml tetracyclin, and then induced for dsRNA production for 4 hours with 1 mM IPTG. *C. elegans* strains were plated onto NGM plates supplemented with 100 µg/ml ampicillin, 10 µg/ml tetracyclin, and 1 mM IPTG and were fed with *E. coli* HT115 pL4440-*coq* to silence *sir-2.1.*

**Motility assays.** 1-5 day old adult hermaphrodites were laid on confluent OP-50-1. The animals were then scored immediately for whole body bends for a duration of 20 seconds as previously described²³, or photos of the tracks were taken using a 2,5X objective on a Zeiss microscope and their amplitudes measured using the Meta View software. 100 animals were scored per experiment and three independent assays performed. Percent rescue of the body bends phenotype was calculated as ((control - test)/control*100) and percent rescue of the sinusoid wave phenotype as ((test - control)/control*100). ANOVA tests and Tukey's Multiple Comparison Tests were used for statistics.

**Lifespan assays.** Fifty synchronized L4 larvae were laid on OP-50.1 bacteria plates (10 animals/plate) at 20°C and transferred to new bacterial plates every other day. Worms were scored every day for normal motility, abnormal motility, paralysis, and death. Three independent assays were performed. The Logrank test (Prism software) was used for the statistical analysis of survival curves.

**Analysis of muscle cells.** Muscle cells in transgenic hermaphrodites were examined for loss of nuclei through counting GFP positive cells. Percent rescue was calculated as ((test - control)/control*100). Muscle cells in transgenic hermaphrodites were examined for PABPN1 expression through fluorescent microscopy. Whole animals were permeabilized and stained with the PABPN1 antiserum VEADa-14 (Centre de Recherche du CHUM, Montreal; 1:50), and fluorescent microscopy was conducted on a Zeiss microscope (Axioplan Imaging II). Observations of the structure of body wall muscles in young adult transgenics after Phalloidin-Rhodamin (FluoProbes) staining were performed as previously described⁵¹. ANOVA tests and Tukey's Multiple Comparison Test were used for statistics.

**Drug assays.** Synchronized populations of L1 larvae expressing GFP and PABPN1 with 10 or 13 Alas were obtained by hypochlorite extraction. To test for muscle nuclei loss, animals were grown in 96-well microtiter plates in 50 µl liquid culture medium with sodium butyrate, resveratrol, sirtinol or splitomicin for 65 hours at 20°C, transferred to agarose pads on slides, and scored for cell nuclei loss at 10X-63X using a Zeiss fluorescence microscope (Axioplan Imaging II). 60 worms were scored per point and three independent assays performed. To test for motility, the synchronized L1 larvae were grown on NGM plates containing drugs until they became 1-5 day old adults. Animals were transferred to new plates every other day to keep them separate from progeny, then transferred to confluent OP-50.1 bacteria test plates and assessed for the number of whole body bends as described above (see Motility assays).

**Western blot analysis.** Worm proteins were prepared using a micro-wave as previously described⁵². Protein extracts were probed using a SIRT1 antibody and actin antibody and revealed using the Western Blot Chemiluminescence reagent Plus kit (GE Healthcare).

### Mammalian cells

**Plasmid constructs.** The cDNAs encoding PABPN1 with 10 or 17 Alas were cloned into the pEGFP-C2 vector, resulting in a fusion of GFP to the N-termini of PABPN1 proteins. The QuikChange™ Site-Directed Mutagenesis Kit (Stratagene, La Jolla, CA) was used to generate the PABPN1-A0 construct. All constructs were verified by DNA sequencing.

**Cell culture and transfection.** Twenty-four hours before transfection, COS-7 cells were seeded in Dulbeco's modified Eagle's medium (DMEM) containing 10% fetal calf serum at a concentration of 2 x 10⁵ cells per well in 12-well plates suited for the automated live microscopy. The cells were transfected with plasmid DNA (1 µg) pre-complexed with the Plus reagent and diluted in Lipofectamine reagent according to the manufacturer's instructions. To check the protein expression before and after the drug treatment, parallel plates were prepared for Western blotting.

**Histone acetylation analysis.** COS-7 cells transfected with normal (10 Alas) and mutant (17 Alas) PABPN1 cDNAs were plated for 48h and treated for the last 24h with 500 µM or 5 mM sodium butyrate. Controls included non-butyrate-treated cells. Cells were lysed in 10 mM Tris, 50 mM sodium bisulfite, 1% triton X-100, 10 mM magnesium chloride, 8.6% sucrose, pH 6.5 and a cocktail of proteases inhibitors for 10 min. Proteins were extracted under acidic conditions as described⁵³. Ten micrograms of whole cell extract were analysed by western blot. Anti-acetylated histone H3 lysine 14 and anti-acetylated histone H4 lysines 5, 8, 12, 16 and anti-histone H1 were used to determine levels of acetylated histones H3 and H4 relative to levels of total histones H1. Signals were quantified using NIH IMAGE 1.62 and t tests used for statistical analysis.

**Drug treatment.** Resveratrol and Sirtinol were dissolved in DMSO. In the initial cell treatment experiments, we performed dose-response experiments, using non-transfected cells, to define the most appropriate doses to be used for the drugs. 0.5 µM resveratrol and 2.5 µM sirtinol were the appropriate doses for drug testing, as they did not cause cell toxicity for the period of experiments (five days), and therefore they were used throughout our study. The drugs or vehicle as a control were applied on transfected cells 24 h post-transfection. Cells (in the 12-well plate) were then incubated for 24 h before analysis using a Leica automated microscope stage.

**Assessment of cell survival.** Cell survival was assessed using live microscopy as previously described¹². Briefly, 12-well plates containing transfected cells treated or not treated with drugs were placed on a live stage microscope equipped with a digital camera, CO₂ incubation chamber, and a motorized X-Y-axis stage and connected to the Openlab software (Improvision). The microscopic robotic stage allows parallel tracking of multiple fields, both within a single well and in multiple wells. Hence, this multimode microscopy approach addresses inter- and intra-sample variability and coherent corrections needed while conducting these experiments. The system was set to memorize three different fields of cells (from each separate well) and control the stage return to the same position at pre-fixed intervals. The microscope allowed us to return back to the same point and count the same cells in the field. Cell survival was monitored every 24 h for five consecutive days, with Day 1 corresponding to 24 h post-transfection before the drug treatment. On average, 150 cells per field per construct were counted. Cell viability was assayed by analysis of GFP positive cells. The average of cell survival of each group of cells was scored for the three different chosen fields and percent rescue calculated as ((test - control)/control*100). Three independent experiments were performed. ANOVA tests and Tukey's Multiple Comparison Test were used for statistics.

**Western blotting.** Cells were harvested at different time points post-transfection and proteins extracted in lysis buffer. Equal amounts of protein were analyzed on 12% SDS-PAGE and transferred to a nitrocellulose membrane. The membranes were probed with a GFP antibody and revealed using the Western Blot Chemiluminescence reagent Plus kit (NEN Life Science Products). Parallel samples were probed with an actin antibody to verify equal loading of lysates.

### RESULTS

### Mutant PABPN1 produces muscle pathology in adult C. elegans

Animals expressing PABPN 1 (with 0, 10, or 13 Alas) and GFP were engineered in the form of two separate proteins under the same promoter Pmyo-3 as GFP::PABPN1 fusion proteins was observed produce some toxicity unrelated to polyAlas, masking the magnitude of polyAla toxicity. *Pmyo-3* is expressed in 104 cells, including all body wall muscle cells, and a few other muscle cells. The expression of integrated arrays encoding mutant PABPN1 specifically produced muscular defects in adults. In culture, *C. elegans* moves by crawling across bacterial lawns in a highly regular sinusoidal pattern, which can be quantified and scored as the number of whole body bends per unit time and amplitude of the sinusoidal wave. Compared to controls, 5-day old PABPN1-A13 animals showed uncoordinated phenotypes including decreased amplitude of the sinusoid wave measured from tracks across a bacterial lawn **(****Fig. 1a****).** This abnormal motility phenotype was accompanied by an increased frequency of body bends in PABPN1-A13 animals but was not observed in PABPN1-A10 animals **(****Fig**.**1b****).** These phenotypes were not due to differences in transgene expression (**Table 1**) or differences in aging rates as all the PABPN1 strains showed the same lifespan (data not shown).

**Table 1: Expression of PABPN1 is unchanged by polyAla length. No difference in PABPN1 expression levels from any of the strains shown (treated or untreated with drugs) was detected by quantitative RT-PCR. NA: not applicable.**

| **Genotype (± treatment)** | **Transgene expression level (**Δ**Ct)** | **P value (T-test)** |
|---|---|---|
| PABPN1-A0 | 7.9 ± 0.7 | NA |
| PABPN1-A10 vs PABPN1-A0 | 8.1 ± 1.3 | 0.87 |
| PABPN1-A13 *vs* PABPN1-A0 | 7.4 ± 1.2 | 0.45 |
| PABPN1-A13; *sir2.1*(geln3) vs PABPN1-A13 | 7.7 ± 1.5 | 0.75 |
| PABPN1-A13 ; *sir2.1*(ok434) vs PABPN1-A13 | 7.6 ± 1.4 | 0.82 |
| PABPN1-A13 ; *daf-16*(mu86) vs PABPN1-A13 | 7.7 ± 1.4 | 0.73 |
| PABPN1-A13 ; *aak-2*(ok524) vs PABPN1-A13 | 7.2 ± 1.1 | 0.79 |
| PABPN1-A13 untreated | 5.0 ± 0.9 | NA |
| PABPN1-A13+ 33 µM butyrate vs untreated | 3.6 ± 0.1 | 0.11 |
| PABPN1-A13+ 33 µM resveratrol vs untreated | 5.2 ± 0.2 | 0.76 |
| PABPN1-A13+ 3.7µM sirtinol vs untreated | 5.2 ± 0.5 | 0.77 |
| PABPN1-A13+ 33 µM splitomicin vs untreated | 4.0 ± 0.3 | 0.22 |

These phenotypes were attributed to human PABPN1 expression as *(i) C. elegans* contains few proteins with polyAlas larger than 10 Alas, and *(ii)* the predicted *C. elegans* ortholog of PABPN1 *pab-3*²⁵ is expressed in the intestine and neurons but not in muscle cells (see gene summary for *pab-3* at http://www.wormbase.org/).

PABPN1 is normally found in the nucleoplasm of muscle cells, forming NIs in muscle fiber nuclei. To test for the localization of human PABPN1 in the transgenic *C. elegans,* we stained whole transgenic worms with a human PABPN1 antiserum. Immunostaining revealed a diffuse expression of PABPN1 with 0, 10, and 13 Alas localized to the nucleoplasm of GFP positive nuclei, with some weak accumulation detected for PABPN1-A13 (**Fig. 1c**). This indicated that mutant PABPN1 not within NIs may produce cytotoxicity, consistent with the notion that the net outcome of NI formation may be muscle protection. Thus, transgenic *C*. *elegans* allows for the study of pathogenic components that may be difficult to study in other models of OPMD pathogenesis and that may occur before, or be concomitant to NI formation.

The appearance of muscle cells was next examined by scoring the number of fluorescent muscle cell nuclei. Compared to GFP animals, the PABPN1-A0 and PABPN1-A10 animals were unaffected whereas PABPN1-A13 animals showed a progressive muscle nuclei loss (MNL) (**Fig. 1d****)**, suggesting the occurrence of degenerative processes. To test for muscle cell degeneration, we further examined muscle cell structure by staining of actin filaments with a rhodamine-phalloidin fluoroprobe. PABPN1-A13 animals showed greater muscle cell defects and cell loss compared to the basal level similarly showed by GFP, PABPN1-A0 and PABPN1-A10 animals (**Fig. 1e**).
Altogether, these data indicated that transgenic nematodes show a progressive muscular pathology produced by soluble to weakly-aggregated mutant PABPN1, providing a useful system to identify genetic and chemical modifiers in vivo.

### Sodium butyrate rescues muscle pathology in nematodes and H4 hypoacetylation in mammalian cells

Besides regulating mRNA processing, human PABPN1 may control the expression of muscle-specific genes⁵. In muscle cells, PABPN1 is associated with SKIP, a protein that binds to the oncoprotein SKI in a complex that regulates transcription⁵. SKIP is a conserved protein that interacts with members of the histone deacetylase (HDAC) complex. It was hypothesized that mutant PABPN1 cytotoxicity may involve altered histone acetylation. To test this hypothesis, PABPN1-10 and PABPN1-A13 worms were incubated with the HDAC inhibitor sodium butyrate. Butyrate showed a strong (the maximal achievable rescue is 28 %) rescue of MNL caused by PABPN1-A13 expression **(****Fig. 2a**). Butyrate treatment did not affect the level of transgene expression (**Table 1**).
To test whether mutant PABPN1 may affect histone acetylation in mammalian cells, acetylation levels of histones H3 and H4 in COS-7 cells transfected with PABPN1 constructs were analysed. The expression of mutant PABPN1 with 17 Alas reduced acetylation of H4, but not H3, compared to normal PABPN1 with 10 Alas **(****Fig. 2b****).** When these cells were treated with butyrate, levels of H4 and H3 acetylation in cells expressing normal or mutant PABPN1 were increased **(****Fig. 2b****).** While acetylation levels were altered, total histone levels, determined by silver staining of proteins and immunostaining of total H1 histones, were unchanged by expressing PABPN1 proteins (**Fig. 2b**)**.** Thus, expression of mutant PABPN1 causes a reduction in acetylation of H4 that is reversed by sodium butyrate, providing insight into the mechanisms for improvement of muscular dystrophy by HDAC inhibitors.

### Mutant PABPN1 cytoxicity is enhanced by increased sir-2.1 dosage through daf-16 and aak-2 activity, and is rescued by sir-2.1, daf-16 and aak-2 deletions

Having shown *C. elegans* allows the effect of deacetylase modulators to be detected on the muscle pathology produced by mt-PABPN1, more investigation was made specifically whether class III deacetylases, also referred to as sirtuins, may be involved in the modulation of mt-PABPN1 cytotoxicity. **Sir2** is a class III HDAC and a major conserved regulator of longevity³⁰. **Sir2** activation has been proposed to protect against neurodegenerative disease-associated proteins, which is dependent on the transcription factor *daf-16*/FoxO in regard to expanded polyglutamines (polyQS)^{18,31}. Unlike what was observed for the expression of mutant polyQs in neurons¹⁸, increased *sir*-*2.1*/SIRT1 dosage enhanced MNL in PABPN1-A13 animals above the basal levels observed in GFP, PABPN1-A0 and PABPN1-A10 strains **(****Fig.** 3a). Consistently, a null mutation of *sir-2.1* or gene knock-down by *sir-2.1* RNAi rescued MNL in PABPN1-A13 animals. The gene knock-down by *sir-2.1* RNAi similarly enhanced MNL in control strains, indicating the rescue of MNL is specific to PABPN1-A13 animals **(****Fig. 3a****).** The reduction of SIR-2.1 expression by *sir-2.1* RNAi was verified by western blotting **(****Figure 5****).** Next, the effects of *daf-16*/FoxO. *daf-16* genetically interacts with *sir.2-1* was investigated in modulating longevity³² and protection against the effects of neurodegenerative disease-associated polypeptides^{18,33}. A null mutation of *daf-16* similarly enhanced MNL in GFP, PABPN1-A0, and PABPN1-A10 strains whereas it rescued MNL in PABPN1-A13 animals **(****Fig. 3a**), suggesting that *daf-16* sustains normal muscle homeostasis and promote alterations produced by mt-PABPN1 expression. Additionally the effects of *aak*-*2*/AMP-activated protein kinase (AMPK) were investigated. A null mutation of *aak-2* rescued MNL in PABPN1-A13 animals with no effect in control strains **(****Fig. 3a**), indicating normal *aak-2* promotes mt-PABPN1 cytotoxicity. Finally, the enhancement of MNL by increased *sir-2.1* dosage in PABPN1-A13 animals was reversed back to rescue by a null mutation of *daf-16* or *aak-2* **(****Fig. 3a**). The effects of genetic manipulations of *sir-2.1, daf-16* and *aak-2* were unrelated to change in transgene expression (**Table 1).** Thus, *sir-2.1* cooperates with *daf-16* and *aak-2* in promoting the muscle pathology produced by mt-PABPN1, with the inhibition of these three genes being beneficial. In regard to behavior and muscle function, *sir-2.1, daf-16* and *aak-2* mutants strongly rescued the abnormal motility (number of body bends) caused by mt-PABPN1 expression (maximal achievable rescue: 37 %), with no effect in control strains **(****Fig. 3b**). Noticeably, the *aak-2* mutant fully rescued abnormal motility as inferred from sinusoid wave amplitudes caused by mt-PABPN1 expression (maximal achievable rescue: 53 %), with no effect in control strains **(****Fig. 3c****).**
Together, these data indicated interactions between normal *sir-2.1, daf-16* and *aak-2* function promote the muscular pathology caused by mutant PABPN1, and strategies to inhibit this gene interaction network may be protective against OPMD pathology.

### Sirtinol and resveratrol show similar profiles of activity in C. elegans and mammalian cells expressing mutant PABPN1

Resveratrol, a plant polyphenol, likely has several intracellular targets and is able to activate sirtuins³⁶. Resveratrol is protective in models of neurodegenerative diseases^{18,37}, metabolic disturbance^{19,20}, and age-decay phenotypes in vertebrates³⁸. Sirtinol is a synthetic Sir2 inhibitor³⁶ whose impact on disease cell survival is not characterized. We hypothesized these two compounds may show opposite effects in modulating muscular pathology in PABPN1-A13 *C. elegans* transgenics. Treating PABPN1-A13 animals with sirtinol resulted in a dose-dependent rescue of MNL **(****Fig. 4a****).** The rescue by sirtinol was dependent on *sir-2.1, daf-16* but not *aak-2* **(****Fig. 4a****).** Sirtinol also rescued abnormal motility in PABPN1-A13 animals with no effect detected in PABPN1-A10 animals, an activity dependent on *sir-2.1* and *daf-16* **(****Fig. 4b****).** The effect of *aak-2* could not be tested as the null mutation of *aak-2* completely rescued the abnormal motility phenotype. Splitomicin, another Sir2 inhibitor³⁹, also rescued the MNL and abnormal motility produced by mt-PABPN 1 expression, with no effect in controls (**Figure 6**). Sirtinol and splitomicin did not affect the level of transgene expression **(Table 1).** In contrast, treatment with resveratrol enhanced MNL in PABPN1-A13 animals, with no effect in PABPN1-A10 animals **(****Fig. 4c****).** Resveratrol did not affect the level of transgene expression **(Table 1).** The enhancement of MNL by resveratrol was mediated by *sir-2.1* and *aak-2* **(****Fig. 4c****).** However, this effect was independent of *daf-16* **(****Fig. 4c**). Resveratrol also showed a *daf-16* independent and *sir-2.1* and *aak-2* dependent activity for the enhancement of abnormal motility produced by mt-PABPN1 expression **(****Fig. 4d**). Together, these data emphasized differences in the mechanisms used by sirtinol and resveratrol, respectively, to modulate the muscular phenotypes produced by mutant PABPN1.

Next, live microscopy was used to examine the effects of sirtinol and resveratrol in COS-7 cells overexpressing GFP-PABPN1 fusion proteins with 0, 10, or 17 Alas. In this model, mutant PABPN1 expression induces greater cell death from day 2 to 6 compared to PABPN1-A10 expression¹² . At day 5, sirtinol strongly enhanced cell survival whereas resveratrol slightly decreased cell survival in mutant PABPN1-A17 cells, the two compounds showing no effects in cells overexpressing GFP-PABPN1 with 0 and 10 Alas **(****Fig.** 4e). Sirtinol or resveratrol treatment did not affect the level of protein expression (**Figure 7**). Thus, the profiles of activity of sirtinol and resveratrol on mutant PABPN1 cytotoxicity in mammalian cells and transgenic nematodes were similar.

Altogether, these data indicated the chemical inhibition of Sir2 protects against muscular dystrophy in OPMD through FoxO-dependent mechanisms whereas compounds able to activate Sir2 like resveratrol are detrimental through mechanisms that involve AMPK.

### DISCUSSION

Our data identify a new mechanism for the modulation of mutant PABPN1 cytotoxicity, namely Sir2 modulation. Several studies have addressed the relationships between NI formation and PABPN1 structure and cytotoxicity^{10,40,41}. The exact significance of NIs in OPMD pathogenesis remains unclear as it may be part of a general defense mechanism against aggregate prone proteins and may not be necessarily toxic¹². This is consistent with the notion initially raised by studies of huntingtin (htt), the HD protein, that NIs may be protective against the cytotoxicity of soluble to poorly aggregated forms of disease proteins and polypeptides like mutant htt, Aβ (a cleavage product of the amyloid precursor protein), and mutant ataxin-1⁴²⁻⁴⁴. Interestingly, the mutant PABPN1 nematodes described herein are indicative of cytotoxicity produced by soluble or poorly-aggregated mutant PABPN1 in the absence of NIs. Thus, while transgenic *C. elegans* do not explain the peculiar muscle-specificity of OPMD as the expression of PABPN1, a ubiquituously-expressed protein in humans, was directed to worm muscles, they provide a unique system for the genetic and chemical manipulation of a toxic component difficult to isolate and study in other in vivo models⁷.
Consistently, we found H4 hypoacetylation is produced by mutant polyAlas in mammalian cells and rescued by sodium butyrate, providing new insight into the role of histone deacetylation in OPMD.

Besides histone acetylation, the acetylation of proteins others than histones, notably transcription factors, may be central to cell decline/survival mechanisms in degenerative disease pathogenesis¹⁸. Proteins at the interface between organismal longevity and survival like the class III deacetylase Sir2/SIRT1 have been associated with neuronal cell protection^{18,31,45,46}. These studies have emphasized neuroprotection by Sir2/SIRT1 activation. In contrast, our data indicate that Sir2 inhibition may protect against OPMD muscle pathology whereas Sir2 activation may be detrimental. Thus, while Sir2 is reported to be a disease target for both neurodegenerative diseases like HD and muscular dystrophies like OPMD, there appears to be a major difference in Sir2 function according to the type of amino-acid homopolymer expansion and/or the cell types affected, with activation being beneficial in polyQ disease neurons and inhibition beneficial in polyAla dystrophic muscles. In both cases, the findings of the present invention indicate that Sir2 genetically interacts with *daf*-*16*/FoxO to elicit cell protection in agreement to what has been shown for the modulation of longevity. The loss of *daf-16* is detrimental to neuronal function in mutant polyQ nematodes¹⁸ and beneficial to muscle protection in mutant PABPN1 nematodes, consistent with the notion that modulation of DAF-16 acetylation and activity by SIR2 may have different outcomes depending on the cell type and stress condition considered⁴⁷. The fact that normal *daf-16* function may promote mutant PABPN1 toxicity in muscles, hence decrease muscle cell survival, is consistent with the notion that an excess of FOXO activity may promote muscle atrophy⁴⁸ and suppress muscle differenciation⁴⁹. Since FOXO acts a stress sensor in the cell⁴⁷, we speculate that FOXO acetylation levels may be higher in neurons expressing mutant polyQs compared to muscles affected by mutant PABPN1, with increased SIR2 activity required to reach the FOXO acetylation levels that activate downstream neuroprotective mechanisms, and decreased SIR2 activity required to achieve an OPMD muscle protection program. Finally, the fact that normal *aak-2* contributes to mutant PABPN1 toxicity and its enhancement by Sir2 activation, together with the previously-reported increase of AMPK by resveratrol in mice on a high-calorie diet¹⁹ and in cultured neurons²¹ suggest that SIR2 and AMPK levels of activity similarly influence cell processes, their increase being detrimental to disease muscles. Interestingly, we observed that a null mutation in *aak-2* enhanced touch receptor neuron dysfunction in *C. elegans* transgenics expressing expanded polyglutamines, which is similar to the effect of a loss-of-function in Sir2. This raises the possibility that while Sir2 and AMPK inhibition may protect OPMD muscles, Sir2 and AMPK activators may protect neurons affected by mutant neurodegenerative proteins.

Consistent with the genetic data, the Sir2 inhibitors sirtinol and splitomicin promoted and the Sir2 activator resveratrol reduced muscle protection in PABPN1 nematodes. Rescue of muscle pathology by sirtinol was dependent on *sir-2.1* and *daf-16* but not *aak-2,* suggesting that FoxO is important for the survival of diseased neurons¹⁸ and muscle cells. The enhancement of muscle pathology by resveratrol was dependent on *sir-2.1, aak-2* and not *daf-16.* This contrasts with our genetic data indicating that the enhancement of mt-PABPN1 cytotoxicity by increased *sir-2.1* dosage is dependent on both *aak-2* and *daf-16,* and is likely due to the multiple targets that may be used by resveratrol to modulate cell response processes. Besides PGC1-∝²⁰, which is not represented in worms, *aak*-*2*/AMPK¹⁹ or ER stress genes⁵⁰ may be involved in mediating the effects of resveratrol in parallel to²¹ or downstream to Sir2. Nonetheless, the effects of *aak-2* loss-of-function used alone or in combination to either increased *sir-2.1* dosage or resveratrol treatment suggest that AMPK inhibitors may ameliorate OPMD pathology, whereas AMPK activators may ameliorate the pathology of neurodegenerative diseases like HD.

Consistent with the profile of activity in PABPN1 nematodes, the Sir2 inhibitor sirtinol promoted and the Sir2 activator resveratrol reduced the survival of mammalian cells overexpressing mutant polyAlas in the context of GFP-PABPN1 fusion proteins. Together with previous finding on resveratrol activity in polyQ nematode neurons and striatal neurons from huntingtin knock-in mice¹⁸, these results further indicate that Sir2 modulators show similar profiles of activity in *C. elegans* and mammalian cells expressing a mutant disease protein.

In conclusion, and in contrast to polyQ expansion and neurodegenerative diseases¹⁸, our genetic data indicate Sir2 inhibition but not activation protects OPMD muscles. Our pharmacological data indicate that (i) Sir2 inhibitors have a profile of activity consistent with the notion that muscle protection is achieved through a reduced activity of Sir2, involving FOXO and not AMPK, and (ii) Sir2 activators known to modulate the activity of other intracellular targets like resveratrol may be toxic through mechanisms involving AMPK. This is consistent with the notion that Sir2/SIRT1 may have several targets essential to cell survival^{19,20} including *daf-16*¹⁸*,* and that the net outcome of FoxOs activity on cell survival and cell apoptosis may greatly depend on the cellular context and the nature of the stress encountered⁴⁷. Finally, our data define Sir2 inhibitors as therapeutics for OPMD and other muscular dystrophies.

## Claims

1. Use of inhibitors of sirtuins and/or AMP-Kinase (AMPK) for the preparation of a medicament for the treatment of polyalanine diseases.

2. Use according to claim 1 of inhibitors of sirtuins for the preparation of a medicament for the treatment of polyalanine diseases.

3. Use according to claim 1 of inhibitors of AMPK for the preparation of a medicament for the treatment of polyalanine diseases.

4. Use according to any of claims claim 1 to 3 **characterized in that** polyalanine disease is chosen in a group comprising oculopharyngeal muscular dystrophy (OPMD), Huntington disease, Congenital central hypoventilation, syndactyly type V, brachydactyly-syndactyly syndrome, X linked mental retardation, X-linked hypopituitarism, Currarino syndrome, lepharophimosis/ptosis/epicanthus inversus syndrome, and schizophrenia.

5. Use according to claim 4 **characterized in that** polyalanine disease is oculopharyngeal muscular dystrophy (OPMD).

6. Use according to claim 4 **characterized in that** polyalanine disease is Huntington disease.

7. Use according to any of claims 1, 2, and 4 to 6 **characterized in that** inhibitors of sirtuins are chosen in a group comprising, cambinol, splitomycin, nicotinamide, suramin, NF279 and NF2030, Dihydrocoumarin, indoles, N,N'-bisbenzylidenebenzene-1,4-diamine and N,N'-bisbenzylidenenaphthalene-1,4-diamine derivatives, in particular N,N'-bis(2-hydroxybenzylidene)benzene-1,4-diamine), 5-{[(2-{[(3,5-dichloro-2-hydroxyphenyl)methylene]amino}phenyl)thio]methyl} -4-(hydroxymethyl)-2-methylpyridin-3-ol, 1,4-di[(4-hydroxyphenethyl)amino]-9,10-dihydroanthracene-9,10-dione, 2-({[3-[(2-hydroxy-3-methoxybenzylidene)amino]-5-(trifluoromethyl)phenyl]imino}methyl)-6-methoxyphenol, 2-chloro-5,5a,6,7,8,9,10,10a-octahydro-Cyclohept[b]indole-6-carboxamide, 3,3'-[[4-[[4-[4-(5,6,7,8-tetrahydro-5,5,8,8-tetramethyl-2-naphthalenyl)-2-thiazolyl]-1-piperidinyl]methyl]phenyl]imino]bis-Propanenitrile, N-[2-(5-fluoro-1H-indol-2-yl)ethyl]-4-[[3-[4-[(4-fluorophenyl)methoxy]phenyl]-1-oxo-2-propen-1-yl]amino]-4-Piperidinecarboxamide, N-[3-[(2-ethylhexyl)oxy]propyl]-4-hydroxy-γ-(4-hydroxyphenyl)-γ-methyl-Benzenebutanamide, sirtinol, and geometrical and/or optical isomers of sirtinol, and siRNA dsRNA ou RNA sirtuins antisens.

8. Use according to claim 7 **characterized in that** inhibitor of sirtuins is sirtinol.

9. Use according to any of claims 1, and 3 to 6 **characterized in that** inhibitors of AMPK are chosen in a group comprising Compound C and indirubin-3'-oxime.

10. Method of identifying a compound which can be useful for the treatment of polyalanine diseases, the method comprising the following steps :
(i) administering a candidate compound inhibitors of sirtuins and/or AMPK to transgenic invertebrate organisms expressing human PABPN1 with 13 Alanines.
(ii) comparing the motility and/or survival of muscle cells of said transgenic invertebrate organisms before and after step (i)
(iii) identifying as compound which can be useful for the treatment of polyalanine diseases compounds that enhances the motility and /or the survival of muscle cells of said transgenic invertebrate organisms.

11. Method according to claim 10 **characterized in that** the method comprises additional step (i') of administering a candidate compound inhibitors of sirtuins and/or AMPK to transgenic invertebrate organisms expressing human PABPN1 with 10 Alanines as a control.

12. Method of identifying a compound which can be useful for the treatment of polyalanine diseases, the method comprising the following steps :
(i) administering a candidate compound inhibitors of sirtuins and/or AMPK to transgenic invertebrate organisms expressing human PABPN1 with 13 Alanines and to transgenic invertebrate organisms expressing human PABPN1 with 10 Alanines.
(ii) comparing the motility and/or survival of muscle cells of transgenic invertebrate organisms expressing human PABPN1 with 13 Alanines, with transgenic invertebrate organisms expressing human PABPN1 with 10 Alanines.
(iii) identifying as compound which can be useful for the treatment of polyalanine diseases compounds that reduces or suppress differences of motility and /or survival of muscle cells of transgenic invertebrate organisms expressing human PABPN1 with 13 Alanines, compared to transgenic invertebrate organisms expressing human PABPN1 with 10 Alanines.


SEQUENCE LISTING

<110> INSTITUT NATIONAL DE LA SANTE ET DE LA RECHERCHE MEDICALE
      (INSERM)

<120> Use of inhibitors of sirtuins and/or ampk for the
      preparation of a medicament for the treatment of
      polyalanine diseases.

<130> D25579

<160> 17

<170> PatentIn version 3.3


<210> 1
<211> 3107
<212> DNA
<213> homo sapiens

<400> 1
aatgaaggtg gacacccaaa tagccccaat acaaatgcct gttcaatcaa ccaaacatct      60
aagcagcaca tctatgtggt agcatattgc caggccgtga gactgcgaat ataaatagga     120
accgcccctc atctgcaggc gctcacaacc tagttagcaa acagtaaaac aattaagcgc     180
gccgtggaca taggcccact tgtcctggga aatgagggga agctggggtt tgcagtggtt     240
tgattgaagg gggactacat gttagaggca cagactgggt gcaggtacac ccaaaggaac     300
gagaagagtg gaaggaaaca acatccacaa agtaaccaca tgctggcgta tcgaaggccg     360
tgatttacgg ttttgagact ttacctcgcc agcaaagggg ggccagtctg ttagcggtgc     420
agattggagg ggtgacattg gaagctgtcc aggaaaaaga aaatggaact ggggagcaga     480
aggcctacgc aagagggcgg gacagacagg acttgtgact agtagctctg gactgaggaa     540
tcctccctgc tttctggtgc gggagagcta gtggatgatg gtgccaataa cctggatggg     600
gaaagtaagc tccctcctgg aatgcttcat tcacaacctc cattttcagc aacatcccat     660
ctactggtgc ttcctggtcg agatacaagt ttcctgaaac tgctgctctg ttttgggcct     720
cacccggcca acagctcact agctggcaag cagtagtatc aagatggcgg ccccctagga     780
ctggctagtc atgtgacctc gggtttccca agtttgaagc ccggcagtcc tttcgggggc     840
aaggttcacc tgtcacgaaa cgagtgtcac cccttcgact ctcgcaagcc aatcggcatc     900
tgagactggg ccactgcggt gaggcgatcg gaagattggt cctttccagt cgcctagcta     960
gggccaatca cggagcgtcc catacttcgc gggcccgccc gtaggccggg gagaagcagg    1020
aatatcgtca cagcgtggcg gtattattac ctaaggactc gataggaggt gggacgcgtg    1080
ttgattgaca ggcagatttc cctaccggga tttgagaatt tggcgcagtg cccgccttag    1140
aggtgcgctt atttgattgc caagtaatat tccccaatgg agtactagct catggtgacg    1200
ggcaggcagc ttgagctaat gagtcctccg tggccggcgc agctctccac atgccgggcg    1260
gcgggcccca gtctgagcgg cgatggcggc ggcggcggcg gcggcagcag cagcgggggc    1320
tgcgggcggt cggggctccg ggccggggcg gcggcgccat cttgtgcccg gggccggtgg    1380
ggaggccggg gaggggcccc cgggggcgcc aggggactac gggaacggcc tggagtctga    1440
ggaactggag cctgaggagc tgctgctgga gcccgagccg gagcccgagc ccgaagagga    1500
gccgccccgg ccccgcgccc ccccgggagc tccgggccct gggcctggtt cgggagcccc    1560
cggcagccaa gaggaggagg aggagccggg actggtcgag ggtgacccgg gggacggcgc    1620
cattgaggac ccggagctgg aagctatcaa agctcgagtc agggagatgg aggaagaagc    1680
tgagaagcta aaggagctac agaacgaggt agagaagcag atgaatatga gtccacctcc    1740
aggcaatgct ggcccggtga tcatgtccat tgaggagaag atggaggctg atgcccgttc    1800
catctatgtt ggcaatgtgg actatggtgc aacagcagaa gagctggaag ctcactttca    1860
tggctgtggt tcagtcaacc gtgttaccat actgtgtgac aaatttagtg gccatcccaa    1920
agggtttgcg tatatagagt tctcagacaa agagtcagtg aggacttcct tggccttaga    1980
tgagtcccta tttagaggaa ggcaaatcaa ggtgatccca aaacgaacca acagaccagg    2040
catcagcaca acagaccggg gttttccacg agcccgctac cgcgcccgga ccaccaacta    2100
caacagctcc cgctctcgat tctacagtgg ttttaacagc aggccccggg gtcgcgtcta    2160
caggggccgg gctagagcga catcatggta ttccccttac taaaaaaagt gtgtattagg    2220
aggagagaga ggaaaaaaag aggaaagaag gaaaaaaaaa agaattaaaa aaaaaaaaaa    2280
gaaaaacaga agatgacctt gatggaaaaa aaatattttt taaaaaaaag atatactgtg    2340

gaagggggga gaatcccata actaactgct gaggagggac ctgctttggg gagtagggga 2400
aggcccaggg agtggggcag ggggctgctt attcactctg gggattcgcc atggacacgt 2460
ctcaactgcg caagctgctt gcccatgttt ccctgccccc ttcaccccct tgggcctgct 2520
caagggtagg tgggcgtggg tggtaggagg gtttttttta cccagggctc tggaaggaca 2580
ccaaactgtt ctgcttgtta ccttccctcc cgtcttctcc tcgcctttca cagtcccctc 2640
ctgcctgctc ctgtccagcc aggtctacca cccaccccac ccctctttct ccggctccct 2700
gcccctccag attgcctggt gatctatttt gtttcctttt gtgtttcttt ttctgttttg 2760
agtgtctttc tttgcaggtt tctgtagccg gaagatctcc gttccgctcc cagcggctcc 2820
agtgtaaatt ccccttcccc ctggggaaat gcactacctt gttttggggg gtttaggggt 2880
gtttttgttt ttcagttgtt ttgttttttt gttttttttt tttcctttgc cttttttccc 2940
ttttatttgg agggaatggg aggaagtggg aacagggagg tgggaggtgg attttgttta 3000
tttttttagc tcatttccag gggtgggaat ttttttttaa tatgtgtcat gaataaagtt 3060
gtttttgaaa ataaaaaaaa aaaaaaaaaa aaaaaaaaaa aaaaaaa 3107

<210> 2
<211> 30
<212> DNA
<213> Artificial sequence

<220>
<223> Forward primer used for amplification of PABPN1 sequence
      with 10 alanines

<400> 2
cgggaattca tggcggcggc ggcggcggcg 30

<210> 3
<211> 39
<212> DNA
<213> Artificial sequence

<220>
<223> Reward primer used for amplification of PABPN1 sequence
      with 13 alanines

<400> 3
cgggaattct tagtaagggg aataccatga tgttgtcgc 39

<210> 4
<211> 22
<212> DNA
<213> Artificial sequence

<220>
<223> Forward primer used for amplification of PABPN1 sequence
      with 0 alanine

<400> 4
cgggaattca tgggggctgc gg 22

<210> 5
<211> 21
<212> DNA
<213> Artificial sequence

<220>
<223> Forward primer for amplification of PABPN1

<400> 5
ggagctggaa gctatcaaag c                                                21

<210> 6
<211> 22
<212> DNA
<213> Artificial sequence

<220>
<223> Reward primer for amplification of PABPN1

<400> 6
ctgttgcacc atagtccaca tt                                               22


<210> 7
<211> 21
<212> DNA
<213> Artificial sequence

<220>
<223> Forward primer for amplification of col-1

<400> 7
catctttgct gaggtcaact a                                                21


<210> 8
<211> 20
<212> DNA
<213> Artificial sequence

<220>
<223> Reward primer for amplification of col-1

<400> 8
catccctcgc attggagatt                                                  20


<210> 9
<211> 747
<212> PRT
<213> homo sapiens

<220>
<223> SIRT1

<400> 9

Met Ala Asp Glu Ala Ala Leu Ala Leu Gln Pro Gly Gly Ser Pro Ser
1               5                   10                  15
Ala Ala Gly Ala Asp Arg Glu Ala Ala Ser Ser Pro Ala Gly Glu Pro
            20                  25                  30
Leu Arg Lys Arg Pro Arg Arg Asp Gly Pro Gly Leu Glu Arg Ser Pro
        35                  40                  45
Gly Glu Pro Gly Gly Ala Ala Pro Glu Arg Glu Val Pro Ala Ala Ala
    50                  55                  60
Arg Gly Cys Pro Gly Ala Ala Ala Ala Ala Leu Trp Arg Glu Ala Glu
65                  70                  75                  80
Ala Glu Ala Ala Ala Ala Gly Gly Glu Gln Glu Ala Gln Ala Thr Ala

85                  90                  95
Ala Ala Gly Glu Gly Asp Asn Gly Pro Gly Leu Gln Gly Pro Ser Arg
            100                 105                 110
Glu Pro Pro Leu Ala Asp Asn Leu Tyr Asp Glu Asp Asp Asp Asp Glu
        115                 120                 125
Gly Glu Glu Glu Glu Glu Ala Ala Ala Ala Ala Ile Gly Tyr Arg Asp
    130                 135                 140
Asn Leu Leu Phe Gly Asp Glu Ile Ile Thr Asn Gly Phe His Ser Cys
145                 150                 155                 160
Glu Ser Asp Glu Glu Asp Arg Ala Ser His Ala Ser Ser Ser Asp Trp
                165                 170                 175
Thr Pro Arg Pro Arg Ile Gly Pro Tyr Thr Phe Val Gln Gln His Leu
            180                 185                 190
Met Ile Gly Thr Asp Pro Arg Thr Ile Leu Lys Asp Leu Leu Pro Glu
        195                 200                 205
Thr Ile Pro Pro Pro Glu Leu Asp Asp Met Thr Leu Trp Gln Ile Val
    210                 215                 220
Ile Asn Ile Leu Ser Glu Pro Pro Lys Arg Lys Lys Arg Lys Asp Ile
225                 230                 235                 240
Asn Thr Ile Glu Asp Ala Val Lys Leu Leu Gln Glu Cys Lys Lys Ile
                245                 250                 255
Ile Val Leu Thr Gly Ala Gly Val Ser Val Ser Cys Gly Ile Pro Asp
            260                 265                 270
Phe Arg Ser Arg Asp Gly Ile Tyr Ala Arg Leu Ala Val Asp Phe Pro
        275                 280                 285
Asp Leu Pro Asp Pro Gln Ala Met Phe Asp Ile Glu Tyr Phe Arg Lys
    290                 295                 300
Asp Pro Arg Pro Phe Phe Lys Phe Ala Lys Glu Ile Tyr Pro Gly Gln
305                 310                 315                 320
Phe Gln Pro Ser Leu Cys His Lys Phe Ile Ala Leu Ser Asp Lys Glu
                325                 330                 335
Gly Lys Leu Leu Arg Asn Tyr Thr Gln Asn Ile Asp Thr Leu Glu Gln
            340                 345                 350
Val Ala Gly Ile Gln Arg Ile Ile Gln Cys His Gly Ser Phe Ala Thr
        355                 360                 365
Ala Ser Cys Leu Ile Cys Lys Tyr Lys Val Asp Cys Glu Ala Val Arg
    370                 375                 380
Gly Asp Ile Phe Asn Gln Val Val Pro Arg Cys Pro Arg Cys Pro Ala
385                 390                 395                 400
Asp Glu Pro Leu Ala Ile Met Lys Pro Glu Ile Val Phe Phe Gly Glu
                405                 410                 415
Asn Leu Pro Glu Gln Phe His Arg Ala Met Lys Tyr Asp Lys Asp Glu
            420                 425                 430
Val Asp Leu Leu Ile Val Ile Gly Ser Ser Leu Lys Val Arg Pro Val
        435                 440                 445
Ala Leu Ile Pro Ser Ser Ile Pro His Glu Val Pro Gln Ile Leu Ile
    450                 455                 460
Asn Arg Glu Pro Leu Pro His Leu His Phe Asp Val Glu Leu Leu Gly
465                 470                 475                 480
Asp Cys Asp Val Ile Ile Asn Glu Leu Cys His Arg Leu Gly Gly Glu
                485                 490                 495
Tyr Ala Lys Leu Cys Cys Asn Pro Val Lys Leu Ser Glu Ile Thr Glu
            500                 505                 510
Lys Pro Pro Arg Thr Gln Lys Glu Leu Ala Tyr Leu Ser Glu Leu Pro
        515                 520                 525
Pro Thr Pro Leu His Val Ser Glu Asp Ser Ser Ser Pro Glu Arg Thr
    530                 535                 540
Ser Pro Pro Asp Ser Ser Val Ile Val Thr Leu Leu Asp Gln Ala Ala
545                 550                 555                 560
Lys Ser Asn Asp Asp Leu Asp Val Ser Glu Ser Lys Gly Cys Met Glu
                565                 570                 575

Glu Lys Pro Gln Glu Val Gln Thr Ser Arg Asn Val Glu Ser Ile Ala
            580                 585                 590
Glu Gln Met Glu Asn Pro Asp Leu Lys Asn Val Gly Ser Ser Thr Gly
        595                 600                 605
Glu Lys Asn Glu Arg Thr Ser Val Ala Gly Thr Val Arg Lys Cys Trp
    610                 615                 620
Pro Asn Arg Val Ala Lys Glu Gln Ile Ser Arg Arg Leu Asp Gly Asn
625                 630                 635                 640
Gln Tyr Leu Phe Leu Pro Pro Asn Arg Tyr Ile Phe His Gly Ala Glu
                645                 650                 655
Val Tyr Ser Asp Ser Glu Asp Asp Val Leu Ser Ser Ser Ser Cys Gly
            660                 665                 670
Ser Asn Ser Asp Ser Gly Thr Cys Gln Ser Pro Ser Leu Glu Glu Pro
        675                 680                 685
Met Glu Asp Glu Ser Glu Ile Glu Glu Phe Tyr Asn Gly Leu Glu Asp
    690                 695                 700
Glu Pro Asp Val Pro Glu Arg Ala Gly Gly Ala Gly Phe Gly Thr Asp
705                 710                 715                 720
Gly Asp Asp Gln Glu Ala Ile Asn Glu Ala Ile Ser Val Lys Gln Glu
                725                 730                 735
Val Thr Asp Met Asn Tyr Pro Ser Asn Lys Ser
            740                 745

<210> 10
<211> 352
<212> PRT
<213> homo sapiens

<220>
<223> SIRT2

<400> 10
Met Asp Phe Leu Arg Asn Leu Phe Ser Gln Thr Leu Ser Leu Gly Ser
1               5                   10                  15
Gln Lys Glu Arg Leu Leu Asp Glu Leu Thr Leu Glu Gly Val Ala Arg
            20                  25                  30
Tyr Met Gln Ser Glu Arg Cys Arg Arg Val Ile Cys Leu Val Gly Ala
        35                  40                  45
Gly Ile Ser Thr Ser Ala Gly Ile Pro Asp Phe Arg Ser Pro Ser Thr
    50                  55                  60
Gly Leu Tyr Asp Asn Leu Glu Lys Tyr His Leu Pro Tyr Pro Glu Ala
65                  70                  75                  80
Ile Phe Glu Ile Ser Tyr Phe Lys Lys His Pro Glu Pro Phe Phe Ala
                85                  90                  95
Leu Ala Lys Glu Leu Tyr Pro Gly Gln Phe Lys Pro Thr Ile Cys His
            100                 105                 110
Tyr Phe Met Arg Leu Leu Lys Asp Lys Gly Leu Leu Leu Arg Cys Tyr
        115                 120                 125
Thr Gln Asn Ile Asp Thr Leu Glu Arg Ile Ala Gly Leu Glu Gln Glu
    130                 135                 140
Asp Leu Val Glu Ala His Gly Thr Phe Tyr Thr Ser His Cys Val Ser
145                 150                 155                 160
Ala Ser Cys Arg His Glu Tyr Pro Leu Ser Trp Met Lys Glu Lys Ile
                165                 170                 175
Phe Ser Glu Val Thr Pro Lys Cys Glu Asp Cys Gln Ser Leu Val Lys
            180                 185                 190
Pro Asp Ile Val Phe Phe Gly Glu Ser Leu Pro Ala Arg Phe Phe Ser
        195                 200                 205
Cys Met Gln Ser Asp Phe Leu Lys Val Asp Leu Leu Leu Val Met Gly
    210                 215                 220

Thr Ser Leu Gln Val Gln Pro Phe Ala Ser Leu Ile Ser Lys Ala Pro
225                 230                 235                 240
Leu Ser Thr Pro Arg Leu Leu Ile Asn Lys Glu Lys Ala Gly Gln Ser
                245                 250                 255
Asp Pro Phe Leu Gly Met Ile Met Gly Leu Gly Gly Gly Met Asp Phe
            260                 265                 270
Asp Ser Lys Lys Ala Tyr Arg Asp Val Ala Trp Leu Gly Glu Cys Asp
        275                 280                 285
Gln Gly Cys Leu Ala Leu Ala Glu Leu Leu Gly Trp Lys Lys Glu Leu
    290                 295                 300
Glu Asp Leu Val Arg Arg Glu His Ala Ser Ile Asp Ala Gln Ser Gly
305                 310                 315                 320
Ala Gly Val Pro Asn Pro Ser Thr Ser Ala Ser Pro Lys Lys Ser Pro
                325                 330                 335
Pro Pro Ala Lys Asp Glu Ala Arg Thr Thr Glu Arg Glu Lys Pro Gln
            340                 345                 350

<210> 11
<211> 399
<212> PRT
<213> homo sapiens

<220>
<223> SIRT3

<400> 11

Met Ala Phe Trp Gly Trp Arg Ala Ala Ala Ala Leu Arg Leu Trp Gly
1               5                   10                  15
Arg Val Val Glu Arg Val Glu Ala Gly Gly Gly Val Gly Pro Phe Gln
            20                  25                  30
Ala Cys Gly Cys Arg Leu Val Leu Gly Gly Arg Asp Asp Val Ser Ala
        35                  40                  45
Gly Leu Arg Gly Ser His Gly Ala Arg Gly Glu Pro Leu Asp Pro Ala
    50                  55                  60
Arg Pro Leu Gln Arg Pro Pro Arg Pro Glu Val Pro Arg Ala Phe Arg
65                  70                  75                  80
Arg Gln Pro Arg Ala Ala Ala Pro Ser Phe Phe Phe Ser Ser Ile Lys
                85                  90                  95
Gly Gly Arg Arg Ser Ile Ser Phe Ser Val Gly Ala Ser Ser Val Val
            100                 105                 110
Gly Ser Gly Gly Ser Ser Asp Lys Gly Lys Leu Ser Leu Gln Asp Val
        115                 120                 125
Ala Glu Leu Ile Arg Ala Arg Ala Cys Gln Arg Val Val Val Met Val
    130                 135                 140
Gly Ala Gly Ile Ser Thr Pro Ser Gly Ile Pro Asp Phe Arg Ser Pro
145                 150                 155                 160
Gly Ser Gly Leu Tyr Ser Asn Leu Gln Gln Tyr Asp Leu Pro Tyr Pro
                165                 170                 175
Glu Ala Ile Phe Glu Leu Pro Phe Phe Phe His Asn Pro Lys Pro Phe
            180                 185                 190
Phe Thr Leu Ala Lys Glu Leu Tyr Pro Gly Asn Tyr Lys Pro Asn Val
        195                 200                 205
Thr His Tyr Phe Leu Arg Leu Leu His Asp Lys Gly Leu Leu Leu Arg
    210                 215                 220
Leu Tyr Thr Gln Asn Ile Asp Gly Leu Glu Arg Val Ser Gly Ile Pro
225                 230                 235                 240
Ala Ser Lys Leu Val Glu Ala His Gly Thr Phe Ala Ser Ala Thr Cys
                245                 250                 255
Thr Val Cys Gln Arg Pro Phe Pro Gly Glu Asp Ile Arg Ala Asp Val

            260                 265                 270
Met Ala Asp Arg Val Pro Arg Cys Pro Val Cys Thr Gly Val Val Lys
        275                 280                 285
Pro Asp Ile Val Phe Phe Gly Glu Pro Leu Pro Gln Arg Phe Leu Leu
    290                 295                 300
His Val Val Asp Phe Pro Met Ala Asp Leu Leu Leu Ile Leu Gly Thr
305                 310                 315                 320
Ser Leu Glu Val Glu Pro Phe Ala Ser Leu Thr Glu Ala Val Arg Ser
                325                 330                 335
Ser Val Pro Arg Leu Leu Ile Asn Arg Asp Leu Val Gly Pro Leu Ala
            340                 345                 350
Trp His Pro Arg Ser Arg Asp Val Ala Gln Leu Gly Asp Val Val His
        355                 360                 365
Gly Val Glu Ser Leu Val Glu Leu Leu Gly Trp Thr Glu Glu Met Arg
    370                 375                 380
Asp Leu Val Gln Arg Glu Thr Gly Lys Leu Asp Gly Pro Asp Lys
385                 390                 395

<210> 12
<211> 314
<212> PRT
<213> homo sapiens

<220>
<223> SIRT4

<400> 12

Met Lys Met Ser Phe Ala Leu Thr Phe Arg Ser Ala Lys Gly Arg Trp
1               5                   10                  15
Ile Ala Asn Pro Ser Gln Pro Cys Ser Lys Ala Ser Ile Gly Leu Phe
            20                  25                  30
Val Pro Ala Ser Pro Pro Leu Asp Pro Glu Lys Val Lys Glu Leu Gln
        35                  40                  45
Arg Phe Ile Thr Leu Ser Lys Arg Leu Leu Val Met Thr Gly Ala Gly
    50                  55                  60
Ile Ser Thr Glu Ser Gly Ile Pro Asp Tyr Arg Ser Glu Lys Val Gly
65                  70                  75                  80
Leu Tyr Ala Arg Thr Asp Arg Arg Pro Ile Gln His Gly Asp Phe Val
                85                  90                  95
Arg Ser Ala Pro Ile Arg Gln Arg Tyr Trp Ala Arg Asn Phe Val Gly
            100                 105                 110
Trp Pro Gln Phe Ser Ser His Gln Pro Asn Pro Ala His Trp Ala Leu
        115                 120                 125
Ser Thr Trp Glu Lys Leu Gly Lys Leu Tyr Trp Leu Val Thr Gln Asn
    130                 135                 140
Val Asp Ala Leu His Thr Lys Ala Gly Ser Arg Arg Leu Thr Glu Leu
145                 150                 155                 160
His Gly Cys Met Asp Arg Val Leu Cys Leu Asp Cys Gly Glu Gln Thr
                165                 170                 175
Pro Arg Gly Val Leu Gln Glu Arg Phe Gln Val Leu Asn Pro Thr Trp
            180                 185                 190
Ser Ala Glu Ala His Gly Leu Ala Pro Asp Gly Asp Val Phe Leu Ser
        195                 200                 205
Glu Glu Gln Val Arg Ser Phe Gln Val Pro Thr Cys Val Gln Cys Gly
    210                 215                 220
Gly His Leu Lys Pro Asp Val Val Phe Phe Gly Asp Thr Val Asn Pro
225                 230                 235                 240
Asp Lys Val Asp Phe Val His Lys Arg Val Lys Glu Ala Asp Ser Leu
                245                 250                 255

Leu Val Val Gly Ser Ser Leu Gln Val Tyr Ser Gly Tyr Arg Phe Ile
            260                 265                 270
Leu Thr Ala Trp Glu Lys Lys Leu Pro Ile Ala Ile Leu Asn Ile Gly
        275                 280                 285
Pro Thr Arg Ser Asp Asp Leu Ala Cys Leu Lys Leu Asn Ser Arg Cys
    290                 295                 300
Gly Glu Leu Leu Pro Leu Ile Asp Pro Cys
305                 310

<210> 13
<211> 310
<212> PRT
<213> homo sapiens

<220>
<223> SIRT5

<400> 13

Met Arg Pro Leu Gln Ile Val Pro Ser Arg Leu Ile Ser Gln Leu Tyr
1               5                   10                  15
Cys Gly Leu Lys Pro Pro Ala Ser Thr Arg Asn Gln Ile Cys Leu Lys
            20                  25                  30
Met Ala Arg Pro Ser Ser Ser Met Ala Asp Phe Arg Lys Phe Phe Ala
        35                  40                  45
Lys Ala Lys His Ile Val Ile Ile Ser Gly Ala Gly Val Ser Ala Glu
    50                  55                  60
Ser Gly Val Pro Thr Phe Arg Gly Ala Gly Gly Tyr Trp Arg Lys Trp
65                  70                  75                  80
Gln Ala Gln Asp Leu Ala Thr Pro Leu Ala Phe Ala His Asn Pro Ser
                85                  90                  95
Arg Val Trp Glu Phe Tyr His Tyr Arg Arg Glu Val Met Gly Ser Lys
            100                 105                 110
Glu Pro Asn Ala Gly His Arg Ala Ile Ala Glu Cys Glu Thr Arg Leu
        115                 120                 125
Gly Lys Gln Gly Arg Arg Val Val Val Ile Thr Gln Asn Ile Asp Glu
    130                 135                 140
Leu His Arg Lys Ala Gly Thr Lys Asn Leu Leu Glu Ile His Gly Ser
145                 150                 155                 160
Leu Phe Lys Thr Arg Cys Thr Ser Cys Gly Val Val Ala Glu Asn Tyr
                165                 170                 175
Lys Ser Pro Ile Cys Pro Ala Leu Ser Gly Lys Gly Ala Pro Glu Pro
            180                 185                 190
Gly Thr Gln Asp Ala Ser Ile Pro Val Glu Lys Leu Pro Arg Cys Glu
        195                 200                 205
Glu Ala Gly Cys Gly Gly Leu Leu Arg Pro His Val Val Trp Phe Gly
    210                 215                 220
Glu Asn Leu Asp Pro Ala Ile Leu Glu Glu Val Asp Arg Glu Leu Ala
225                 230                 235                 240
His Cys Asp Leu Cys Leu Val Val Gly Thr Ser Ser Val Val Tyr Pro
                245                 250                 255
Ala Ala Met Phe Ala Pro Gln Val Ala Ala Arg Gly Val Pro Val Ala
            260                 265                 270
Glu Phe Asn Thr Glu Thr Thr Pro Ala Thr Asn Arg Phe Arg Phe His
        275                 280                 285
Phe Gln Gly Pro Cys Gly Thr Thr Leu Pro Glu Ala Leu Ala Cys His
    290                 295                 300
Glu Asn Glu Thr Val Ser
305                 310

<210> 14
<211> 355
<212> PRT
<213> homo sapiens

<220>
<223> SIRT6

<400> 14


<210> 15
<211> 400

<212> PRT
<213> homo sapiens

<220>
<223> SIRT7

<400> 15

Met Ala Ala Gly Gly Leu Ser Arg Ser Glu Arg Lys Ala Ala Glu Arg
1               5                   10                  15
Val Arg Arg Leu Arg Glu Glu Gln Gln Arg Glu Arg Leu Arg Gln Val
            20                  25                  30
Ser Arg Ile Leu Arg Lys Ala Ala Ala Glu Arg Ser Ala Glu Glu Gly
        35                  40                  45
Arg Leu Leu Ala Glu Ser Ala Asp Leu Val Thr Glu Leu Gln Gly Arg
    50                  55                  60
Ser Arg Arg Arg Glu Gly Leu Lys Arg Arg Gln Glu Glu Val Cys Asp
65                  70                  75                  80
Asp Pro Glu Glu Leu Arg Gly Lys Val Arg Glu Leu Ala Ser Ala Val
                85                  90                  95
Arg Asn Ala Lys Tyr Leu Val Val Tyr Thr Gly Ala Gly Ile Ser Thr
            100                 105                 110
Ala Ala Ser Ile Pro Asp Tyr Arg Gly Pro Asn Gly Val Trp Thr Leu
        115                 120                 125
Leu Gln Lys Gly Arg Ser Val Ser Ala Ala Asp Leu Ser Glu Ala Glu
    130                 135                 140
Pro Thr Leu Thr His Met Ser Ile Thr Arg Leu His Glu Gln Lys Leu
145                 150                 155                 160
Val Gln His Val Val Ser Gln Asn Cys Asp Gly Leu His Leu Arg Ser
                165                 170                 175
Gly Leu Pro Arg Thr Ala Ile Ser Glu Leu His Gly Asn Met Tyr Ile
            180                 185                 190
Glu Val Cys Thr Ser Cys Val Pro Asn Arg Glu Tyr Val Arg Val Phe
        195                 200                 205
Asp Val Thr Glu Arg Thr Ala Leu His Arg His Gln Thr Gly Arg Thr
    210                 215                 220
Cys His Lys Cys Gly Thr Gln Leu Arg Asp Thr Ile Val His Phe Gly
225                 230                 235                 240
Glu Arg Gly Thr Leu Gly Gln Pro Leu Asn Trp Glu Ala Ala Thr Glu
                245                 250                 255
Ala Ala Ser Arg Ala Asp Thr Ile Leu Cys Leu Gly Ser Ser Leu Lys
            260                 265                 270
Val Leu Lys Lys Tyr Pro Arg Leu Trp Cys Met Thr Lys Pro Pro Ser
        275                 280                 285
Arg Arg Pro Lys Leu Tyr Ile Val Asn Leu Gln Trp Thr Pro Lys Asp
    290                 295                 300
Asp Trp Ala Ala Leu Lys Leu His Gly Lys Cys Asp Asp Val Met Arg
305                 310                 315                 320
Leu Leu Met Ala Glu Leu Gly Leu Glu Ile Pro Ala Tyr Ser Arg Trp
                325                 330                 335
Gln Asp Pro Ile Phe Ser Leu Ala Thr Pro Leu Arg Ala Gly Glu Glu
            340                 345                 350
Gly Ser His Ser Arg Lys Ser Leu Cys Arg Ser Arg Glu Glu Ala Pro
        355                 360                 365
Pro Gly Asp Arg Gly Ala Pro Leu Ser Ser Ala Pro Ile Leu Gly Gly
    370                 375                 380
Trp Phe Gly Arg Gly Cys Thr Lys Arg Thr Lys Arg Lys Lys Val Thr
385                 390                 395                 400

<210> 16

<211> 21
<212> RNA
<213> Artificial sequence

<220>
<223> SiRNA

<400> 16
acuuugcugu aacccuguat t                                                21


<210> 17
<211> 21
<212> RNA
<213> Artificial sequence

<220>
<223> SiRNA

<400> 17
ttugaaacga cauugggaca u                                                21

SEQUENCE LISTING

<110> INSTITUT NATIONAL DE LA SANTE ET DE LA RECHERCHE MEDICALE
      (INSERM)

<120> Use of inhibitors of sirtuins and/or ampk for the
      preparation of a medicament for the treatment of
      polyalanine diseases.

<130> D25579

<160> 17

<170> PatentIn version 3.3


<210> 1
<211> 3107
<212> DNA
<213> homo sapiens

<400> 1
aatgaaggtg gacacccaaa tagccccaat acaaatgcct gttcaatcaa ccaaacatct      60
aagcagcaca tctatgtggt agcatattgc caggccgtga gactgcgaat ataaatagga     120
accgcccctc atctgcaggc gctcacaacc tagttagcaa acagtaaaac aattaagcgc     180
gccgtggaca taggcccact tgtcctggga aatgagggga agctggggtt tgcagtggtt     240
tgattgaagg gggactacat gttagaggca cagactgggt gcaggtacac ccaaaggaac     300
gagaagagtg gaaggaaaca acatccacaa agtaaccaca tgctggcgta tcgaaggccg     360
tgatttacgg ttttgagact ttacctcgcc agcaaagggg ggccagtctg ttagcggtgc     420
agattggagg ggtgacattg gaagctgtcc aggaaaaaga aaatggaact ggggagcaga     480
aggcctacgc aagagggcgg gacagacagg acttgtgact agtagctctg gactgaggaa     540
tcctccctgc tttctggtgc gggagagcta gtggatgatg gtgccaataa cctggatggg     600
gaaagtaagc tccctcctgg aatgcttcat tcacaacctc cattttcagc aacatcccat     660
ctactggtgc ttcctggtcg agatacaagt ttcctgaaac tgctgctctg ttttgggcct     720
cacccggcca acagctcact agctggcaag cagtagtatc aagatggcgg ccccctagga     780
ctggctagtc atgtgacctc gggtttccca agtttgaagc ccggcagtcc tttcgggggc     840
aaggttcacc tgtcacgaaa cgagtgtcac cccttcgact ctcgcaagcc aatcggcatc     900
tgagactggg ccactgcggt gaggcgatcg gaagattggt cctttccagt cgcctagcta     960
gggccaatca cggagcgtcc catacttcgc gggcccgccc gtaggccggg gagaagcagg    1020
aatatcgtca cagcgtggcg gtattattac ctaaggactc gataggaggt gggacgcgtg    1080
ttgattgaca ggcagatttc cctaccggga tttgagaatt tggcgcagtg cccgccttag    1140
aggtgcgctt atttgattgc caagtaatat tccccaatgg agtactagct catggtgacg    1200
ggcaggcagc ttgagctaat gagtcctccg tggccggcgc agctctccac atgccgggcg    1260
gcgggcccca gtctgagcgg cgatggcggc ggcggcggcg gcggcagcag cagcgggggc    1320
tgcgggcggt cggggctccg ggccggggcg gcggcgccat cttgtgcccg gggccggtgg    1380
ggaggccggg gagggggccc cggggggcgc aggggactac gggaacggcc tggagtctga    1440
ggaactggag cctgaggagc tgctgctgga gcccgagccg gagcccgagc ccgaagagga    1500
gccgccccgg ccccgcgccc ccccgggagc tccgggccct gggcctggtt cgggagcccc    1560
cggcagccaa gaggaggagg aggagccggg actggtcgag ggtgacccgg gggacggcgc    1620
cattgaggac ccggagctgg aagctatcaa agctcgagtc agggagatgg aggaagaagc    1680
tgagaagcta aaggagctac agaacgaggt agagaagcag atgaatatga gtccacctcc    1740
aggcaatgct ggcccggtga tcatgtccat tgaggagaag atggaggctg atgcccgttc    1800
catctatgtt ggcaatgtgg actatggtgc aacagcagaa gagctggaag ctcactttca    1860
tggctgtggt tcagtcaacc gtgttaccat actgtgtgac aaatttagtg gccatcccaa    1920
agggtttgcg tatatagagt tctcagacaa agagtcagtg aggacttcct tggccttaga    1980
tgagtcccta tttagaggaa ggcaaatcaa ggtgatccca aaacgaacca acagaccagg    2040
catcagcaca acagaccggg gttttccacg agcccgctac cgcgcccgga ccaccaacta    2100
caacagctcc cgctctcgat tctacagtgg ttttaacagc aggccccggg gtcgcgtcta    2160
caggggccgg gctagagcga catcatggta ttccccttac taaaaaaagt gtgtattagg    2220
aggagagaga ggaaaaaaag aggaaagaag gaaaaaaaaa agaattaaaa aaaaaaaaaa    2280
gaaaaacaga agatgacctt gatggaaaaa aaatattttt taaaaaaaag atatactgtg    2340

gaagggggga gaatcccata actaactgct gaggagggac ctgctttggg gagtagggga   2400
aggcccaggg agtggggcag ggggctgctt attcactctg gggattcgcc atggacacgt   2460
ctcaactgcg caagctgctt gcccatgttt ccctgccccc ttcaccccct tgggcctgct   2520
caagggtagg tgggcgtggg tggtaggagg gtttttttta cccagggctc tggaaggaca   2580
ccaaactgtt ctgcttgtta ccttccctcc cgtcttctcc tcgcctttca cagtcccctc   2640
ctgcctgctc ctgtccagcc aggtctacca cccaccccac ccctctttct ccggctccct   2700
gcccctccag attgcctggt gatctatttt gtttcctttt gtgtttcttt ttctgttttg   2760
agtgtctttc tttgcaggtt tctgtagccg gaagatctcc gttccgctcc cagcggctcc   2820
agtgtaaatt ccccttcccc ctggggaaat gcactacctt gttttggggg gtttaggggt   2880
gtttttgttt ttcagttgtt ttgttttttt gttttttttt tttcctttgc ctttttcccc   2940
ttttatttgg agggaatggg aggaagtggg aacagggagg tgggaggtgg attttgttta   3000
tttttttagc tcatttccag gggtgggaat ttttttttaa tatgtgtcat gaataaagtt   3060
gtttttgaaa ataaaaaaaa aaaaaaaaaa aaaaaaaaaa aaaaaaa                3107

<210> 2
<211> 30
<212> DNA
<213> Artificial sequence

<220>
<223> Forward primer used for amplification of PABPN1 sequence
      with 10 alanines

<400> 2
cgggaattca tggcggcggc ggcggcggcg                                    30

<210> 3
<211> 39
<212> DNA
<213> Artificial sequence

<220>
<223> Reward primer used for amplification of PABPN1 sequence
      with 13 alanines

<400> 3
cgggaattct tagtaagggg aataccatga tgttgtcgc                          39

<210> 4
<211> 22
<212> DNA
<213> Artificial sequence

<220>
<223> Forward primer used for amplification of PABPN1 sequence
      with 0 alanine

<400> 4
cgggaattca tgggggctgc gg                                            22

<210> 5
<211> 21
<212> DNA
<213> Artificial sequence

<220>
<223> Forward primer for amplification of PABPN1

<400> 5
ggagctggaa gctatcaaag c                                                21

<210> 6
<211> 22
<212> DNA
<213> Artificial sequence

<220>
<223> Reward primer for amplification of PABPN1

<400> 6
ctgttgcacc atagtccaca tt                                               22


<210> 7
<211> 21
<212> DNA
<213> Artificial sequence

<220>
<223> Forward primer for amplification of col-1

<400> 7
catctttgct gaggtcaact a                                                21


<210> 8
<211> 20
<212> DNA
<213> Artificial sequence

<220>
<223> Reward primer for amplification of col-1

<400> 8
catccctcgc attggagatt                                                  20


<210> 9
<211> 747
<212> PRT
<213> homo sapiens

<220>
<223> SIRT1

<400> 9

Met Ala Asp Glu Ala Ala Leu Ala Leu Gln Pro Gly Gly Ser Pro Ser
1               5                   10                  15
Ala Ala Gly Ala Asp Arg Glu Ala Ala Ser Ser Pro Ala Gly Glu Pro
            20                  25                  30
Leu Arg Lys Arg Pro Arg Arg Asp Gly Pro Gly Leu Glu Arg Ser Pro
        35                  40                  45
Gly Glu Pro Gly Gly Ala Ala Pro Glu Arg Glu Val Pro Ala Ala Ala
    50                  55                  60
Arg Gly Cys Pro Gly Ala Ala Ala Ala Ala Leu Trp Arg Glu Ala Glu
65                  70                  75                  80
Ala Glu Ala Ala Ala Ala Gly Gly Glu Gln Glu Ala Gln Ala Thr Ala

                85                  90                  95
Ala Ala Gly Glu Gly Asp Asn Gly Pro Gly Leu Gln Gly Pro Ser Arg
            100                 105                 110
Glu Pro Pro Leu Ala Asp Asn Leu Tyr Asp Glu Asp Asp Asp Asp Glu
        115                 120                 125
Gly Glu Glu Glu Glu Glu Ala Ala Ala Ala Ala Ile Gly Tyr Arg Asp
    130                 135                 140
Asn Leu Leu Phe Gly Asp Glu Ile Ile Thr Asn Gly Phe His Ser Cys
145                 150                 155                 160
Glu Ser Asp Glu Glu Asp Arg Ala Ser His Ala Ser Ser Ser Asp Trp
                165                 170                 175
Thr Pro Arg Pro Arg Ile Gly Pro Tyr Thr Phe Val Gln Gln His Leu
            180                 185                 190
Met Ile Gly Thr Asp Pro Arg Thr Ile Leu Lys Asp Leu Leu Pro Glu
        195                 200                 205
Thr Ile Pro Pro Pro Glu Leu Asp Asp Met Thr Leu Trp Gln Ile Val
    210                 215                 220
Ile Asn Ile Leu Ser Glu Pro Pro Lys Arg Lys Lys Arg Lys Asp Ile
225                 230                 235                 240
Asn Thr Ile Glu Asp Ala Val Lys Leu Leu Gln Glu Cys Lys Lys Ile
                245                 250                 255
Ile Val Leu Thr Gly Ala Gly Val Ser Val Ser Cys Gly Ile Pro Asp
            260                 265                 270
Phe Arg Ser Arg Asp Gly Ile Tyr Ala Arg Leu Ala Val Asp Phe Pro
        275                 280                 285
Asp Leu Pro Asp Pro Gln Ala Met Phe Asp Ile Glu Tyr Phe Arg Lys
    290                 295                 300
Asp Pro Arg Pro Phe Phe Lys Phe Ala Lys Glu Ile Tyr Pro Gly Gln
305                 310                 315                 320
Phe Gln Pro Ser Leu Cys His Lys Phe Ile Ala Leu Ser Asp Lys Glu
                325                 330                 335
Gly Lys Leu Leu Arg Asn Tyr Thr Gln Asn Ile Asp Thr Leu Glu Gln
            340                 345                 350
Val Ala Gly Ile Gln Arg Ile Ile Gln Cys His Gly Ser Phe Ala Thr
        355                 360                 365
Ala Ser Cys Leu Ile Cys Lys Tyr Lys Val Asp Cys Glu Ala Val Arg
    370                 375                 380
Gly Asp Ile Phe Asn Gln Val Val Pro Arg Cys Pro Arg Cys Pro Ala
385                 390                 395                 400
Asp Glu Pro Leu Ala Ile Met Lys Pro Glu Ile Val Phe Phe Gly Glu
                405                 410                 415
Asn Leu Pro Glu Gln Phe His Arg Ala Met Lys Tyr Asp Lys Asp Glu
            420                 425                 430
Val Asp Leu Leu Ile Val Ile Gly Ser Ser Leu Lys Val Arg Pro Val
        435                 440                 445
Ala Leu Ile Pro Ser Ser Ile Pro His Glu Val Pro Gln Ile Leu Ile
    450                 455                 460
Asn Arg Glu Pro Leu Pro His Leu His Phe Asp Val Glu Leu Leu Gly
465                 470                 475                 480
Asp Cys Asp Val Ile Ile Asn Glu Leu Cys His Arg Leu Gly Gly Glu
                485                 490                 495
Tyr Ala Lys Leu Cys Cys Asn Pro Val Lys Leu Ser Glu Ile Thr Glu
            500                 505                 510
Lys Pro Pro Arg Thr Gln Lys Glu Leu Ala Tyr Leu Ser Glu Leu Pro
        515                 520                 525
Pro Thr Pro Leu His Val Ser Glu Asp Ser Ser Ser Pro Glu Arg Thr
    530                 535                 540
Ser Pro Pro Asp Ser Ser Val Ile Val Thr Leu Leu Asp Gln Ala Ala
545                 550                 555                 560
Lys Ser Asn Asp Asp Leu Asp Val Ser Glu Ser Lys Gly Cys Met Glu
                565                 570                 575

Glu Lys Pro Gln Glu Val Gln Thr Ser Arg Asn Val Glu Ser Ile Ala
                580                 585                 590
Glu Gln Met Glu Asn Pro Asp Leu Lys Asn Val Gly Ser Ser Thr Gly
        595                 600                 605
Glu Lys Asn Glu Arg Thr Ser Val Ala Gly Thr Val Arg Lys Cys Trp
    610                 615                 620
Pro Asn Arg Val Ala Lys Glu Gln Ile Ser Arg Arg Leu Asp Gly Asn
625                 630                 635                 640
Gln Tyr Leu Phe Leu Pro Pro Asn Arg Tyr Ile Phe His Gly Ala Glu
                645                 650                 655
Val Tyr Ser Asp Ser Glu Asp Asp Val Leu Ser Ser Ser Ser Cys Gly
            660                 665                 670
Ser Asn Ser Asp Ser Gly Thr Cys Gln Ser Pro Ser Leu Glu Glu Pro
        675                 680                 685
Met Glu Asp Glu Ser Glu Ile Glu Glu Phe Tyr Asn Gly Leu Glu Asp
    690                 695                 700
Glu Pro Asp Val Pro Glu Arg Ala Gly Gly Ala Gly Phe Gly Thr Asp
705                 710                 715                 720
Gly Asp Asp Gln Glu Ala Ile Asn Glu Ala Ile Ser Val Lys Gln Glu
                725                 730                 735
Val Thr Asp Met Asn Tyr Pro Ser Asn Lys Ser
            740                 745

<210> 10
<211> 352
<212> PRT
<213> homo sapiens

<220>
<223> SIRT2

<400> 10
Met Asp Phe Leu Arg Asn Leu Phe Ser Gln Thr Leu Ser Leu Gly Ser
1               5                   10                  15
Gln Lys Glu Arg Leu Leu Asp Glu Leu Thr Leu Glu Gly Val Ala Arg
            20                  25                  30
Tyr Met Gln Ser Glu Arg Cys Arg Arg Val Ile Cys Leu Val Gly Ala
        35                  40                  45
Gly Ile Ser Thr Ser Ala Gly Ile Pro Asp Phe Arg Ser Pro Ser Thr
    50                  55                  60
Gly Leu Tyr Asp Asn Leu Glu Lys Tyr His Leu Pro Tyr Pro Glu Ala
65                  70                  75                  80
Ile Phe Glu Ile Ser Tyr Phe Lys Lys His Pro Glu Pro Phe Phe Ala
                85                  90                  95
Leu Ala Lys Glu Leu Tyr Pro Gly Gln Phe Lys Pro Thr Ile Cys His
            100                 105                 110
Tyr Phe Met Arg Leu Leu Lys Asp Lys Gly Leu Leu Leu Arg Cys Tyr
        115                 120                 125
Thr Gln Asn Ile Asp Thr Leu Glu Arg Ile Ala Gly Leu Glu Gln Glu
    130                 135                 140
Asp Leu Val Glu Ala His Gly Thr Phe Tyr Thr Ser His Cys Val Ser
145                 150                 155                 160
Ala Ser Cys Arg His Glu Tyr Pro Leu Ser Trp Met Lys Glu Lys Ile
                165                 170                 175
Phe Ser Glu Val Thr Pro Lys Cys Glu Asp Cys Gln Ser Leu Val Lys
            180                 185                 190
Pro Asp Ile Val Phe Phe Gly Glu Ser Leu Pro Ala Arg Phe Phe Ser
        195                 200                 205
Cys Met Gln Ser Asp Phe Leu Lys Val Asp Leu Leu Leu Val Met Gly
    210                 215                 220

Thr Ser Leu Gln Val Gln Pro Phe Ala Ser Leu Ile Ser Lys Ala Pro
225                 230                 235                 240
Leu Ser Thr Pro Arg Leu Leu Ile Asn Lys Glu Lys Ala Gly Gln Ser
                245                 250                 255
Asp Pro Phe Leu Gly Met Ile Met Gly Leu Gly Gly Gly Met Asp Phe
            260                 265                 270
Asp Ser Lys Lys Ala Tyr Arg Asp Val Ala Trp Leu Gly Glu Cys Asp
        275                 280                 285
Gln Gly Cys Leu Ala Leu Ala Glu Leu Leu Gly Trp Lys Lys Glu Leu
    290                 295                 300
Glu Asp Leu Val Arg Arg Glu His Ala Ser Ile Asp Ala Gln Ser Gly
305                 310                 315                 320
Ala Gly Val Pro Asn Pro Ser Thr Ser Ala Ser Pro Lys Lys Ser Pro
                325                 330                 335
Pro Pro Ala Lys Asp Glu Ala Arg Thr Thr Glu Arg Glu Lys Pro Gln
            340                 345                 350

<210> 11
<211> 399
<212> PRT
<213> homo sapiens

<220>
<223> SIRT3

<400> 11

Met Ala Phe Trp Gly Trp Arg Ala Ala Ala Ala Leu Arg Leu Trp Gly
1               5                   10                  15
Arg Val Val Glu Arg Val Glu Ala Gly Gly Gly Val Gly Pro Phe Gln
            20                  25                  30
Ala Cys Gly Cys Arg Leu Val Leu Gly Gly Arg Asp Asp Val Ser Ala
        35                  40                  45
Gly Leu Arg Gly Ser His Gly Ala Arg Gly Glu Pro Leu Asp Pro Ala
    50                  55                  60
Arg Pro Leu Gln Arg Pro Pro Arg Pro Glu Val Pro Arg Ala Phe Arg
65                  70                  75                  80
Arg Gln Pro Arg Ala Ala Ala Pro Ser Phe Phe Phe Ser Ser Ile Lys
                85                  90                  95
Gly Gly Arg Arg Ser Ile Ser Phe Ser Val Gly Ala Ser Ser Val Val
            100                 105                 110
Gly Ser Gly Gly Ser Ser Asp Lys Gly Lys Leu Ser Leu Gln Asp Val
        115                 120                 125
Ala Glu Leu Ile Arg Ala Arg Ala Cys Gln Arg Val Val Val Met Val
    130                 135                 140
Gly Ala Gly Ile Ser Thr Pro Ser Gly Ile Pro Asp Phe Arg Ser Pro
145                 150                 155                 160
Gly Ser Gly Leu Tyr Ser Asn Leu Gln Gln Tyr Asp Leu Pro Tyr Pro
                165                 170                 175
Glu Ala Ile Phe Glu Leu Pro Phe Phe Phe His Asn Pro Lys Pro Phe
            180                 185                 190
Phe Thr Leu Ala Lys Glu Leu Tyr Pro Gly Asn Tyr Lys Pro Asn Val
        195                 200                 205
Thr His Tyr Phe Leu Arg Leu Leu His Asp Lys Gly Leu Leu Leu Arg
    210                 215                 220
Leu Tyr Thr Gln Asn Ile Asp Gly Leu Glu Arg Val Ser Gly Ile Pro
225                 230                 235                 240
Ala Ser Lys Leu Val Glu Ala His Gly Thr Phe Ala Ser Ala Thr Cys
                245                 250                 255
Thr Val Cys Gln Arg Pro Phe Pro Gly Glu Asp Ile Arg Ala Asp Val

260                 265                 270
Met Ala Asp Arg Val Pro Arg Cys Pro Val Cys Thr Gly Val Val Lys
        275                 280                 285
Pro Asp Ile Val Phe Phe Gly Glu Pro Leu Pro Gln Arg Phe Leu Leu
    290                 295                 300
His Val Val Asp Phe Pro Met Ala Asp Leu Leu Leu Ile Leu Gly Thr
305                 310                 315                 320
Ser Leu Glu Val Glu Pro Phe Ala Ser Leu Thr Glu Ala Val Arg Ser
                325                 330                 335
Ser Val Pro Arg Leu Leu Ile Asn Arg Asp Leu Val Gly Pro Leu Ala
            340                 345                 350
Trp His Pro Arg Ser Arg Asp Val Ala Gln Leu Gly Asp Val Val His
        355                 360                 365
Gly Val Glu Ser Leu Val Glu Leu Leu Gly Trp Thr Glu Glu Met Arg
    370                 375                 380
Asp Leu Val Gln Arg Glu Thr Gly Lys Leu Asp Gly Pro Asp Lys
385                 390                 395

<210> 12
<211> 314
<212> PRT
<213> homo sapiens

<220>
<223> SIRT4

<400> 12

Met Lys Met Ser Phe Ala Leu Thr Phe Arg Ser Ala Lys Gly Arg Trp
1               5                   10                  15
Ile Ala Asn Pro Ser Gln Pro Cys Ser Lys Ala Ser Ile Gly Leu Phe
            20                  25                  30
Val Pro Ala Ser Pro Pro Leu Asp Pro Glu Lys Val Lys Glu Leu Gln
        35                  40                  45
Arg Phe Ile Thr Leu Ser Lys Arg Leu Leu Val Met Thr Gly Ala Gly
    50                  55                  60
Ile Ser Thr Glu Ser Gly Ile Pro Asp Tyr Arg Ser Glu Lys Val Gly
65                  70                  75                  80
Leu Tyr Ala Arg Thr Asp Arg Arg Pro Ile Gln His Gly Asp Phe Val
                85                  90                  95
Arg Ser Ala Pro Ile Arg Gln Arg Tyr Trp Ala Arg Asn Phe Val Gly
            100                 105                 110
Trp Pro Gln Phe Ser Ser His Gln Pro Asn Pro Ala His Trp Ala Leu
        115                 120                 125
Ser Thr Trp Glu Lys Leu Gly Lys Leu Tyr Trp Leu Val Thr Gln Asn
    130                 135                 140
Val Asp Ala Leu His Thr Lys Ala Gly Ser Arg Arg Leu Thr Glu Leu
145                 150                 155                 160
His Gly Cys Met Asp Arg Val Leu Cys Leu Asp Cys Gly Glu Gln Thr
                165                 170                 175
Pro Arg Gly Val Leu Gln Glu Arg Phe Gln Val Leu Asn Pro Thr Trp
            180                 185                 190
Ser Ala Glu Ala His Gly Leu Ala Pro Asp Gly Asp Val Phe Leu Ser
        195                 200                 205
Glu Glu Gln Val Arg Ser Phe Gln Val Pro Thr Cys Val Gln Cys Gly
    210                 215                 220
Gly His Leu Lys Pro Asp Val Val Phe Phe Gly Asp Thr Val Asn Pro
225                 230                 235                 240
Asp Lys Val Asp Phe Val His Lys Arg Val Lys Glu Ala Asp Ser Leu
                245                 250                 255

Leu Val Val Gly Ser Ser Leu Gln Val Tyr Ser Gly Tyr Arg Phe Ile
            260                 265                 270
Leu Thr Ala Trp Glu Lys Lys Leu Pro Ile Ala Ile Leu Asn Ile Gly
        275                 280                 285
Pro Thr Arg Ser Asp Asp Leu Ala Cys Leu Lys Leu Asn Ser Arg Cys
    290                 295                 300
Gly Glu Leu Leu Pro Leu Ile Asp Pro Cys
305                 310

<210> 13
<211> 310
<212> PRT
<213> homo sapiens

<220>
<223> SIRT5

<400> 13

Met Arg Pro Leu Gln Ile Val Pro Ser Arg Leu Ile Ser Gln Leu Tyr
1               5                   10                  15
Cys Gly Leu Lys Pro Pro Ala Ser Thr Arg Asn Gln Ile Cys Leu Lys
            20                  25                  30
Met Ala Arg Pro Ser Ser Ser Met Ala Asp Phe Arg Lys Phe Phe Ala
        35                  40                  45
Lys Ala Lys His Ile Val Ile Ile Ser Gly Ala Gly Val Ser Ala Glu
    50                  55                  60
Ser Gly Val Pro Thr Phe Arg Gly Ala Gly Gly Tyr Trp Arg Lys Trp
65                  70                  75                  80
Gln Ala Gln Asp Leu Ala Thr Pro Leu Ala Phe Ala His Asn Pro Ser
                85                  90                  95
Arg Val Trp Glu Phe Tyr His Tyr Arg Arg Glu Val Met Gly Ser Lys
            100                 105                 110
Glu Pro Asn Ala Gly His Arg Ala Ile Ala Glu Cys Glu Thr Arg Leu
        115                 120                 125
Gly Lys Gln Gly Arg Arg Val Val Val Ile Thr Gln Asn Ile Asp Glu
    130                 135                 140
Leu His Arg Lys Ala Gly Thr Lys Asn Leu Leu Glu Ile His Gly Ser
145                 150                 155                 160
Leu Phe Lys Thr Arg Cys Thr Ser Cys Gly Val Val Ala Glu Asn Tyr
                165                 170                 175
Lys Ser Pro Ile Cys Pro Ala Leu Ser Gly Lys Gly Ala Pro Glu Pro
            180                 185                 190
Gly Thr Gln Asp Ala Ser Ile Pro Val Glu Lys Leu Pro Arg Cys Glu
        195                 200                 205
Glu Ala Gly Cys Gly Gly Leu Leu Arg Pro His Val Val Trp Phe Gly
    210                 215                 220
Glu Asn Leu Asp Pro Ala Ile Leu Glu Glu Val Asp Arg Glu Leu Ala
225                 230                 235                 240
His Cys Asp Leu Cys Leu Val Val Gly Thr Ser Ser Val Val Tyr Pro
                245                 250                 255
Ala Ala Met Phe Ala Pro Gln Val Ala Ala Arg Gly Val Pro Val Ala
            260                 265                 270
Glu Phe Asn Thr Glu Thr Thr Pro Ala Thr Asn Arg Phe Arg Phe His
        275                 280                 285
Phe Gln Gly Pro Cys Gly Thr Thr Leu Pro Glu Ala Leu Ala Cys His
    290                 295                 300
Glu Asn Glu Thr Val Ser
305                 310

<210> 14
<211> 355
<212> PRT
<213> homo sapiens

<220>
<223> SIRT6

<400> 14


<210> 15
<211> 400

<212> PRT
<213> homo sapiens

<220>
<223> SIRT7

<400> 15

Met Ala Ala Gly Gly Leu Ser Arg Ser Glu Arg Lys Ala Ala Glu Arg
1               5                   10                  15
Val Arg Arg Leu Arg Glu Glu Gln Gln Arg Glu Arg Leu Arg Gln Val
            20                  25                  30
Ser Arg Ile Leu Arg Lys Ala Ala Ala Glu Arg Ser Ala Glu Glu Gly
        35                  40                  45
Arg Leu Leu Ala Glu Ser Ala Asp Leu Val Thr Glu Leu Gln Gly Arg
    50                  55                  60
Ser Arg Arg Arg Glu Gly Leu Lys Arg Arg Gln Glu Glu Val Cys Asp
65                  70                  75                  80
Asp Pro Glu Glu Leu Arg Gly Lys Val Arg Glu Leu Ala Ser Ala Val
                85                  90                  95
Arg Asn Ala Lys Tyr Leu Val Val Tyr Thr Gly Ala Gly Ile Ser Thr
            100                 105                 110
Ala Ala Ser Ile Pro Asp Tyr Arg Gly Pro Asn Gly Val Trp Thr Leu
        115                 120                 125
Leu Gln Lys Gly Arg Ser Val Ser Ala Ala Asp Leu Ser Glu Ala Glu
    130                 135                 140
Pro Thr Leu Thr His Met Ser Ile Thr Arg Leu His Glu Gln Lys Leu
145                 150                 155                 160
Val Gln His Val Val Ser Gln Asn Cys Asp Gly Leu His Leu Arg Ser
                165                 170                 175
Gly Leu Pro Arg Thr Ala Ile Ser Glu Leu His Gly Asn Met Tyr Ile
            180                 185                 190
Glu Val Cys Thr Ser Cys Val Pro Asn Arg Glu Tyr Val Arg Val Phe
        195                 200                 205
Asp Val Thr Glu Arg Thr Ala Leu His Arg His Gln Thr Gly Arg Thr
    210                 215                 220
Cys His Lys Cys Gly Thr Gln Leu Arg Asp Thr Ile Val His Phe Gly
225                 230                 235                 240
Glu Arg Gly Thr Leu Gly Gln Pro Leu Asn Trp Glu Ala Ala Thr Glu
                245                 250                 255
Ala Ala Ser Arg Ala Asp Thr Ile Leu Cys Leu Gly Ser Ser Leu Lys
            260                 265                 270
Val Leu Lys Lys Tyr Pro Arg Leu Trp Cys Met Thr Lys Pro Pro Ser
        275                 280                 285
Arg Arg Pro Lys Leu Tyr Ile Val Asn Leu Gln Trp Thr Pro Lys Asp
    290                 295                 300
Asp Trp Ala Ala Leu Lys Leu His Gly Lys Cys Asp Asp Val Met Arg
305                 310                 315                 320
Leu Leu Met Ala Glu Leu Gly Leu Glu Ile Pro Ala Tyr Ser Arg Trp
                325                 330                 335
Gln Asp Pro Ile Phe Ser Leu Ala Thr Pro Leu Arg Ala Gly Glu Glu
            340                 345                 350
Gly Ser His Ser Arg Lys Ser Leu Cys Arg Ser Arg Glu Glu Ala Pro
        355                 360                 365
Pro Gly Asp Arg Gly Ala Pro Leu Ser Ser Ala Pro Ile Leu Gly Gly
    370                 375                 380
Trp Phe Gly Arg Gly Cys Thr Lys Arg Thr Lys Arg Lys Lys Val Thr
385                 390                 395                 400


<210> 16

<211> 21
<212> RNA
<213> Artificial sequence

<220>
<223> SiRNA
<220>
<221> misc_feature
<222> (20)..(21)
<223> n represente a Thymine

<400> 16
acuuugcugu aacccuguan n                                                21


<210> 17
<211> 21
<212> RNA
<213> Artificial sequence

<220>
<223> SiRNA
<220>
<221> misc_feature
<222> (1)..(2)
<223> n represent a thymine

<400> 17
nnugaaacga cauugggaca u                                                21